# EUROPEAN PATENT APPLICATION

(11) **EP 4 659 687 A1**
(43) Date of publication of application: **10.12.2025**
(21) Application number: 24180500.1
(22) Date of filing: 06.06.2024
(51) Int. Cl.: A61B 17/12, A61F 2/24

(54) **ELONGATE WIRE IMPLANT BETWEEN THERAPEUTIC IMPLANT AND NATIVE TISSUE FOR PREVENTING BLOOD LEAKAGE WITHIN HEART AND ASSOCIATED DEVICES, SYSTEMS, AND METHODS**

(71) Applicant: Koninklijke Philips N.V., 5656 AG Eindhoven (NL)
(72) Inventor: MCPEAK, Thomas John, Eindhoven (NL)
(74) Representative: Philips Intellectual Property & Standards

(57) **Abstract**

Devices, systems, and methods for preventing leakage through a therapeutic implant are provided. An exemplary apparatus includes an elongate wire configured to be implanted within a heart of a patient such that the elongate wire is positioned within a non-circular gap between a therapeutic implant positioned within the heart and a native heart tissue of the patient to stop a leakage of blood through the non-circular gap. The elongate wire comprises a single length of material set in a serpentine shape. Moreover, the elongate wire is configured to retain the serpentine shape when the elongate wire is positioned within the non-circular gap.

## Description

### TECHNICAL FIELD

The subject matter described herein relates to stopping leakage around therapeutic implants and, in particular, leakage around therapeutic cardiac implants such as replacement valves and occlusion devices. For example, an elongate wire implant can be catheter-deployed to stop such leaks by filling gaps between the therapeutic implant and the anatomy.

### BACKGROUND

Various types of therapeutic implants may be implanted into the heart to treat a variety of heart conditions. For example, a heart valve may be damaged or diseased such that blood flow does not flow properly therethrough. In some cases, the valve may allow blood to flow in the opposite direction from normal blood flow (i.e. regurgitation) or significantly alter the blood flow in the direction of normal blood flow. In other cases, there may be stenosis of the valve that prevents sufficient blood flow therethrough. Thus, the existing valve may be removed and/or trimmed and a mechanical, bioprosthetic, or tissue-engineered replacement valve may be implanted in place of the existing valve. The replacement valve may be designed to replace the existing, natural valve to provide normal blood flow through the heart.

In another example, for patients with diseases like atrial fibrillation, there may be a high risk of developing blood clots in the left atrial appendage (LAA). Thus, an occlusion device may be implanted at the opening of the LAA to prevent blood flow therein, thereby minimizing the risk of clots developing in the LAA.

However, when therapeutic implants such as these are implanted into a patient, there may be leakage between the exterior of the therapeutic implant and the wall of the anatomy of the heart. When a therapeutic implant is deployed in the heart, the circumference of the implant may not fully oppose the wall of the anatomy, resulting in one or more gaps between the implant and the wall. Undesirable blood flow may move through these gaps (i.e. leakage), resulting in lower performance of the implant. For example, in some cases, the therapeutic implant may not expand to the full size of the opening. This may occur because therapeutic implants are generally packaged and sold in discrete sizes, which are not tailored to the patient's specific anatomy. Thus, these gaps may form when an implant smaller than the size of the anatomy is deployed at the treatment site. In other cases, the therapeutic implant may have a circular shape and the anatomy has an ovular or non-circular shape. Thus, gaps may be created where the circumference of the circular-shaped plug and the non-circular shaped anatomy are not aligned. In either of these cases, the gaps between the therapeutic implant and the anatomy are generally non-circular and may be a variety of shapes, including crescent-shaped.

Thus, after deploying the therapeutic implant, it may be desirable (or necessary) to plug or fill these gaps to prevent leakage. Current methods of treating these gaps use circular or rectangular devices or plugs. However, because the gaps are often non-circular and non-rectangular, the circular or rectangular plugs may not sufficiently plug the gap to prevent blood flow therethrough.

Moreover, many of the devices currently used to plug gaps are made from Nitinol wire braids with a polyester fabric fill that are heat-set to shape. These devices have a screw thread on a proximal end that threadedly couples to a threaded actuator. Thus, only one of these devices can be delivered at a time, making them expensive and time-consuming to implant.

The information included in this Introduction section of the specification, including any references cited herein and any description or discussion thereof, is included for context and/or technical reference purposes only and is not to be regarded as subject matter by which the scope of the disclosure is to be bound or otherwise limited in any manner.

### SUMMARY

Aspects of the present disclosure are directed to a leak filling device, with associated systems, and a method to treat leakage between a therapeutic device and the neighboring tissue using one or more elongate wires that have been pre-loaded into a single delivery system. The device includes a catheter-based deployment device with an elongate wire disposed therein. The elongate wire may be set (e.g. heat-set) into a serpentine shape that curves between a plurality of peaks and a plurality of troughs. The serpentine shape may make the elongate wire flexible or resilient so that it is biased to return to its heat-set serpentine shape when deformed or constrained. The wire delivery catheter may include a notch or longitudinal slit extending proximally from a distal end thereof. A push rod may be disposed within the catheter such that it engages a proximal-facing end of the elongate wire within the catheter. The catheter can be placed at a gap between a therapeutic device and a wall of the heart (e.g. location of a heart valve, LAA). The push rod can then be moved distally to push the elongate wire out of the notch on the distal end of the catheter. The notch allows the elongate wire to be deployed at an angle relative to the longitudinal axis of the catheter (e.g. perpendicular or at an oblique angle to the longitudinal axis). The elongate wire may be deployed parallel to the gap such that the serpentine shape is oriented so that the peaks and troughs contact and/or press against the therapeutic device and the tissue on either side of the gap. The elongate wire may be deployed perpendicular to the gap such that the serpentine shape is oriented so that the peaks and troughs point up and down within gap. The elongate wire may be constrained within the gap so that it fits to the shape of the gap to fill it and prevent leakage. Moreover, because the elongate wire may be inserted into the gap under tension, the tension may hold the elongate wire within the gap and press against the ends of the gap (e.g. like a compression spring) to narrow or close the gap. Thus, the present disclosure advantageously provides an implantable elongate wire that can sufficiently close gaps of all shapes and sizes, including non-circular gaps. Moreover, because a push rod is used to push against the elongate wire to push it out of the catheter, a coupling element (such as a threaded coupling) between the push rod and elongate wire is not necessary, reducing cost and allowing multiple wires or lengths of wire to be inserted using a single catheter delivery system.

In an exemplary aspect, an apparatus is provided. The apparatus includes an elongate wire configured to be implanted within a heart of a patient such that the elongate wire is positioned within a non-circular gap between a therapeutic implant positioned within the heart and a native heart tissue of the patient to stop a leakage of blood through the non-circular gap. The elongate wire comprises a single length of material set in a serpentine shape. Moreover, the elongate wire is configured to retain the serpentine shape when the elongate wire is positioned within the non-circular gap.

In one aspect, the serpentine shape includes a plurality of peaks and a plurality of troughs. When a first length of the elongate wire is positioned within the non-circular gap, the serpentine shape has a first state with a first distance between at least one of the plurality of peaks or the plurality of troughs, where the first state has an uncompressed state or a relatively less compressed state. In one aspect, when a greater, second length of the elongate wire is positioned within the non-circular gap, the serpentine shape has a second state with a smaller, second distance between at least one of the plurality of peaks or the plurality of troughs, where the second state has a compressed state. In one aspect, when the elongate wire is positioned within the non-circular gap, the elongate wire is configured to have compression in a longitudinal direction of the serpentine shape. In one aspect, the compression of the serpentine shape is configured to cause the elongate wire to be under tension when the elongate wire is positioned within the non-circular gap. In one aspect, the elongate wire includes at least one of fibers or barbs configured to facilitate endothelization.

In one aspect, the apparatus further includes a delivery catheter including a catheter wall defining a lumen configured to receive the elongate wire before the elongate wire is positioned within the non-circular gap. The apparatus further includes a push rod configured to be positioned within the lumen, contact the elongate wire within the lumen, and have distal movement causing the elongate wire to exit the delivery catheter. In one aspect, the delivery catheter has a longitudinal axis, a proximal portion, and a distal portion. The distal portion includes a longitudinal slit on a side of the catheter wall and the serpentine shape is configured to cause the elongate wire to exit the delivery catheter via the longitudinal slit at an angle relative to the longitudinal axis. In one aspect, the serpentine shape is configured to such that contact between the elongate wire and at least one of the therapeutic implant or the native heart tissue is configured to urge the delivery catheter to move from a first end of the non-circular gap to a second end of the non-circular gap. In one aspect, the therapeutic implant includes a prosthetic valve such that the gap includes a paravalvular leakage or an occlusion device such that the gap includes an occlusion leakage.

In an exemplary aspect, a system for treating leakage between an implanted therapeutic device and tissue of a patient is provided. The system includes a catheter and an implant. The catheter includes a lumen and a distal portion. The distal portion includes a notch. The implant includes a wire having a serpentine shape along at least a part of a length of the wire. The implant is disposed within the lumen of the catheter. The implant is configured to exit the lumen via the notch at the distal portion of the catheter.

In one aspect, the serpentine shape of the wire includes a plurality of peaks and a plurality of troughs. In one aspect, each peak of the plurality of peaks is spaced from neighboring troughs of the plurality of troughs in an x-dimension and a y-dimension, each peak of the plurality of peaks is spaced from neighboring peaks along the x-dimension, and each trough of the plurality of troughs is spaced from neighboring peaks along the x-dimension. In one aspect, the plurality of peaks and the plurality of troughs are aligned in a z-dimension. In one aspect, the plurality of peaks and the plurality of troughs are not aligned in a z-dimension.

In one aspect, the implant further includes at least one of a plurality of barbs or a plurality of fibers disposed on at least a portion of the wire. In one aspect, the wire is compressible via the serpentine shape such that the wire is compressed within the lumen of the catheter. In one aspect, the notch extends proximally from a distal end of the distal portion of the catheter. In one aspect, the notch extends from an outer surface of the catheter to the lumen. In one aspect, the system further includes a push rod configured to move the implant distally within the lumen such that the implant exits the lumen through the notch in the distal portion.

This Summary is provided to introduce a selection of concepts in a simplified form that are further described below in the Detailed Description. This Summary is not intended to identify key features or essential features of the claimed subject matter, nor is it intended to limit the scope of the claimed subject matter. A more extensive presentation of features, details, utilities, and advantages of aspects of the present disclosure, e.g., as defined in the claims, is provided in the following written description of various examples and/or aspects of the disclosure and illustrated in the accompanying drawings.

### BRIEF DESCRIPTION OF THE DRAWINGS

Illustrative aspects of the present disclosure will be described with reference to the accompanying drawings, of which:
Fig. 1A is a side view of a human heart according to aspects of the present disclosure.
Fig. 1B is a cross-sectional side view of a human heart according to aspects of the present disclosure.
Fig. 2 is a cross-sectional side view of an aortic valve replacement in a human heart according to aspects of the present disclosure.
Fig. 3 is a diagrammatic, cross-sectional top view of an aortic valve replacement of a degenerated natural aortic valve by a transvenous/transcatheter aortic valve repair (TAVR), according to aspects of the present disclosure.
Fig. 4A is a diagrammatic top view of a replacement valve positioned within the heart, in the open position, according to aspects of the present disclosure.
Fig. 4B is a diagrammatic top view of the replacement valve of Fig. 4A in the closed position, according to aspects of the present disclosure.
Fig. 5A is a diagrammatic cross-sectional side view of a replacement valve in an open position, according to aspects of the present disclosure.
Fig. 5B is a diagrammatic cross-sectional side views of a replacement valve in a closed position, according to aspects of the present disclosure.
Fig. 6 is a cross-sectional view of an occlusion device implanted in the left atrial appendage (LAA), according to aspects of the present disclosure.
Fig. 7A is a diagrammatic cross-sectional view of the occlusion device along section line 7-7 in Fig. 6, according to aspects of the present disclosure.
Fig. 7B is a diagrammatic cross-sectional view of the LAA along section line 7-7 in Fig. 6, according to aspects of the present disclosure.
Fig. 7C is a diagrammatic cross-sectional view of the occlusion device deployed within the LAA, according to aspects of the present disclosure.
Fig. 8 is a schematic, diagrammatic view of a system according to aspects of the present disclosure.
Fig. 9 is a diagrammatic front view of an elongate wire, according to aspects of the present disclosure.
Fig. 10A is a diagrammatic front view of an exemplary elongate wire, according to aspects of the present disclosure.
Fig. 10B is a diagrammatic front view of another exemplary elongate wire, according to aspects of the present disclosure.
Fig. 11A is a diagrammatic side view of an exemplary elongate wire, according to aspects of the present disclosure.
Fig. 11B is a diagrammatic side view of an exemplary elongate wire, according to aspects of the present disclosure.
Fig. 11C is a diagrammatic perspective side view of the elongate wire shown in Fig. 11B, according to aspects of the present disclosure.
Fig. 12A is a diagrammatic front view of a wire delivery catheter, according to aspects of the present disclosure.
Fig. 12B is a diagrammatic side view of a wire delivery catheter, according to aspects of the present disclosure.
Figs 13A-13B are diagrammatic cross-sectional views of an exemplary wire delivery catheter, according to aspects of the present disclosure.
Figs 14A-14B are diagrammatic cross-sectional views of another exemplary wire delivery catheter, according to aspects of the present disclosure.
Fig. 15 illustrates a diagrammatic cross-sectional view of a wire delivery catheter with a push rod disposed therein, according to aspects of the present disclosure.
Fig. 16 illustrates a diagrammatic cross-sectional view of a wire delivery catheter with a push rod and elongate wire disposed therein, according to aspects of the present disclosure.
Fig. 17A illustrates a diagrammatic cross-sectional view of an exemplary wire delivery catheter with a push rod and an elongate wire partially disposed therein, according to aspects of the present disclosure.
Fig. 17B illustrates a diagrammatic cross-sectional view of another exemplary wire delivery catheter with a push rod and an elongate wire partially disposed therein, according to aspects of the present disclosure.
Fig. 18 is a diagrammatic top view of the deployment of the elongate wire within a non-circular gap between a therapeutic implant and the neighboring tissue, according to aspects of the present disclosure.
Fig. 19 is a diagrammatic top view of the continued deployment of the elongate wire within a non-circular gap between a therapeutic implant and the neighboring tissue, according to aspects of the present disclosure.
Fig. 20 is a diagrammatic top view of various positions of a wire delivery catheter during deployment of an elongate wire within a non-circular gap between a therapeutic implant and the neighboring tissue, according to aspects of the present disclosure.
Fig. 21A is a diagrammatic top view of an example of an elongate wire inserted into a non-circular gap between a therapeutic implant and the neighboring tissue, according to aspects of the present disclosure.
Fig. 21B is a diagrammatic top view of another example of an elongate wire inserted into a non-circular gap between a therapeutic implant and the neighboring tissue, according to aspects of the present disclosure.
Fig. 22A is a diagrammatic top view of another example of an elongate wire inserted into a non-circular gap between a therapeutic implant and the neighboring tissue just after insertion, according to aspects of the present disclosure.
Fig. 22B is a diagrammatic top view of another example of an elongate wire inserted into a non-circular gap between a therapeutic implant and the neighboring tissue a period of time after insertion, according to aspects of the present disclosure.
Figs 23A-23D are diagrammatic views of a series of steps in a method for delivering and deploying an elongate wire using an elongate wire delivery catheter and a push rod, according to aspects of the present disclosure.
Fig. 24A is a diagrammatic cross-sectional side view of the replacement valve with an elongate wire implanted, according to aspects of the present disclosure.
Fig. 24B is a diagrammatic cross-sectional side view of the replacement valve with an elongate wire implanted, according to aspects of the present disclosure.
Fig. 25 illustrates an exemplary wire delivery catheter with a cutter and a push rod and an elongate wire partially disposed therein, according to aspects of the present disclosure.
Fig. 26 is a schematic diagram of a processor circuit, according to aspects of the present disclosure.

### DETAILED DESCRIPTION

For the purposes of promoting an understanding of the principles of the present disclosure, reference will now be made to the examples illustrated in the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates. In particular, it is fully contemplated that the features, components, and/or steps described with respect to one example and/or aspect may be combined with the features, components, and/or steps described with respect to other examples and/or aspects of the present disclosure. Additionally, while the description below may refer to blood vessels, it will be understood that the present disclosure is not limited to such applications. For example, the devices, systems, and methods described herein may be used in any body chamber or body lumen, including an esophagus, veins, arteries, intestines, ventricles, atria, or any other body lumen and/or chamber. For the sake of brevity, however, the numerous iterations of these combinations will not be described separately.

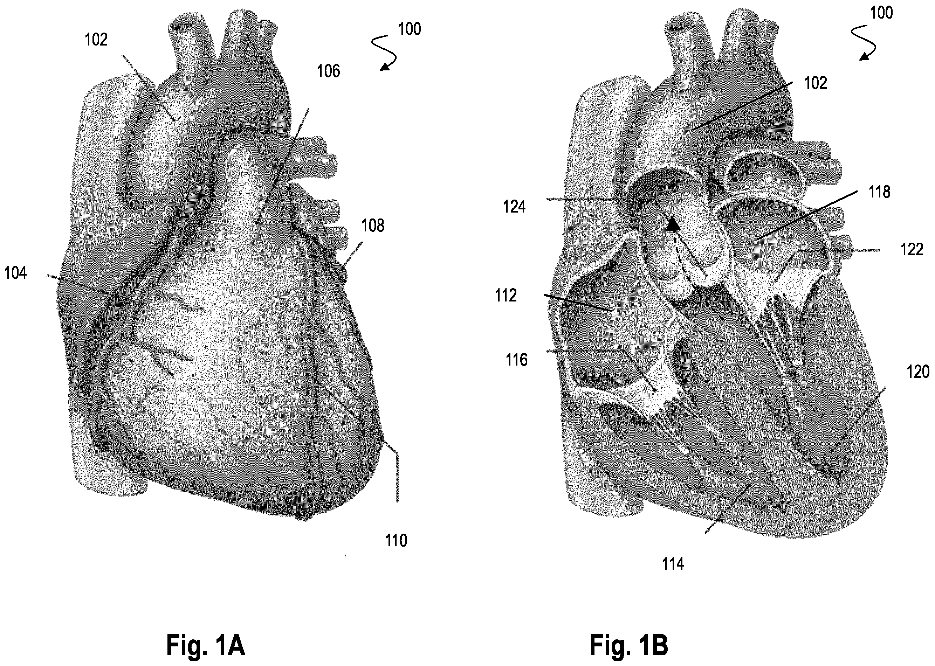

Fig. 1A is a side view of a human heart 100 according to aspects of the present disclosure. Visible are an aorta 102 from which stems a right coronary artery 104 and a left main coronary artery 106. The left main coronary artery 106 branches into a left circumflex coronary artery 108 and a left anterior descending coronary artery 110. The right coronary artery 104, the left main coronary artery 106, the left circumflex coronary artery 108, and a left anterior descending coronary artery 110 are the arteries that provide oxygen-rich blood to muscles of the human heart 100.

Fig. 1B is a cross-sectional side view of a human heart 100 according to aspects of the present disclosure. Visible are a right atrium 112 and a right ventricle 114. In that regard, oxygen-poor blood enters the human heart 100 in the right atrium 112 and travels to the right ventricle 114 through the tricuspid valve 116. The oxygen-poor blood leaves the right ventricle 114 and travels to the lungs. Also visible are a left atrium 118 and a left ventricle 120. In that regard, oxygen-rich blood is received from the lungs in the left atrium 118 and travels to the left ventricle 120 through the mitral valve 122. The oxygen-rich blood leaves the left ventricle 120 and goes out to the body through the aorta 102 via an aortic valve 124.

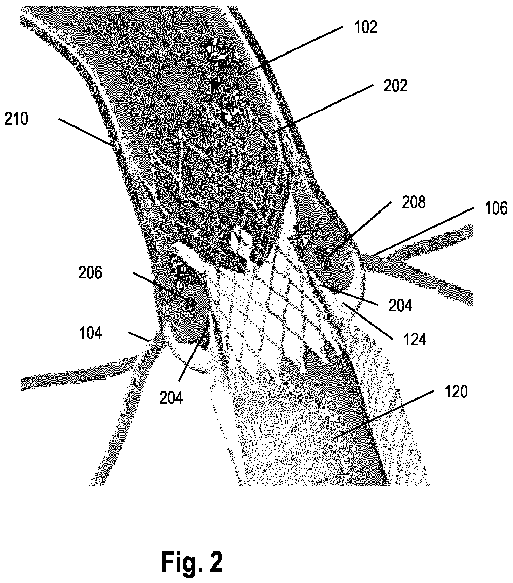

Fig. 2 is a cross-sectional side view of an aortic valve 124 replacement in a human heart 100 according to aspects of the present disclosure. In some aspects, e.g., when aortic valve stenosis has occurred to the aortic valve 124 that keeps blood flowing in the correct direction from the left ventricle 120 to the aorta 102, a transvenous/transcatheter aortic valve repair (TAVR) procedure may be performed to replace the natural aortic valve 124 with a replacement aortic valve 202. In some instances, portions of one or more leaflets 204 of the natural aortic valve 124 may be resected so that openings 206 and 208 to the right coronary artery 104 and the left main coronary artery 106, respectively, so that, when the one or more leaflets 204 are pressed against the natural heart wall 210, the openings 206 and 208 remain unobstructed so that oxygen-rich blood may flow to the muscles of the human heart. The natural heart wall 210 may be a natural aorta wall, a natural heart chamber wall, or a natural aortic valve wall.

The TAVR procedure is shown here for exemplary purposes only; it is understood that other heart valves and heart valve replacement procedure types may generate paravalvular leaks that require plugging, and thus fall within the scope of the present disclosure.

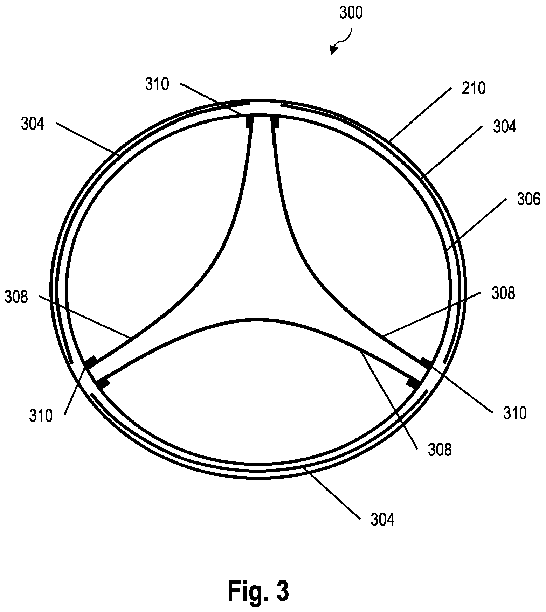

Fig. 3 is a diagrammatic, cross-sectional top view of an aortic valve replacement 300 of a degenerated natural aortic valve utilizing a TAVR valve, according to aspects of the present disclosure. Visible are the natural heart wall 210, the natural aortic valve leaflets 304, the TAVR valve wall 306, the TAVR valve leaflets 308, and the TAVR commissural tabs 310. The TAVR valve leaflets 308 may for example be constructed from bovine animal tissue coupled to the wire frame of the TAVR valve wall 306 via the commissural tabs 310. When inserted, the TAVR valve pushes the remaining unresected portions of the natural aortic valve leaflets 304 against the natural heart wall 210 such that the natural aortic valve leaflets 304 are pinned and/or secured between an outside of the TAVR valve wall 306 and the natural heart wall 210.

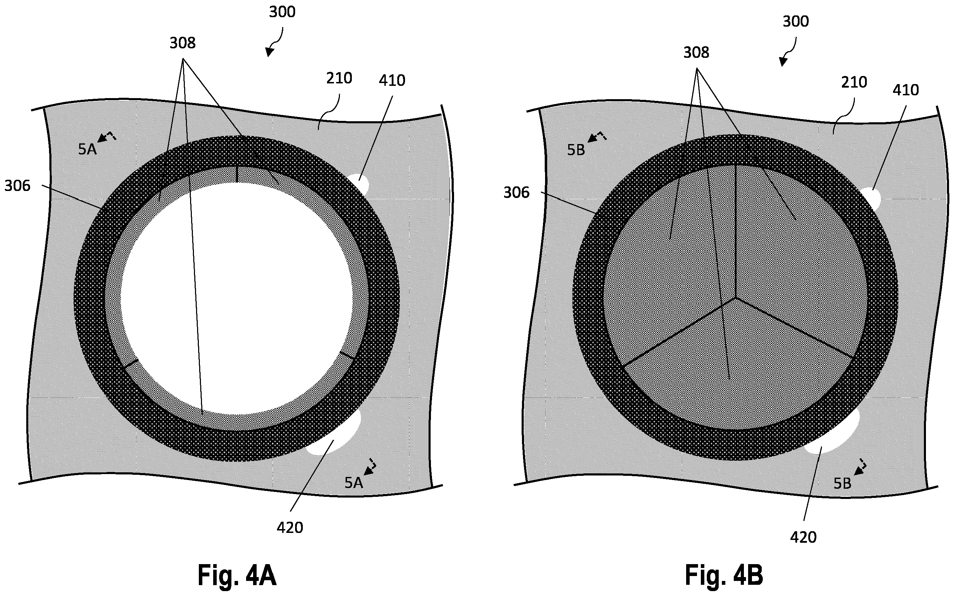

Fig. 4A is a diagrammatic top view of a replacement valve 300 (e.g., a prosthetic, mechanical, or donor valve) positioned within the heart, in the open position, according to aspects of the present disclosure. The leaflets 308 of the replacement valve periodically open (to allow blood flow in desired direction) and close (to prevent blood flow in opposite undesired direction) during heart cycle. Artificial leaflets are one example of a mechanism that open/closes to periodically allow/stop blood flow, but a mechanical/prosthetic valve or donor valve can have any suitable mechanism that open/closes to periodically allow/stop blood flow.

In the example shown in Fig. 4A, the replacement valve wall 306 is joined to the natural heart wall 210, but it is imperfect because of a relatively smaller paravalvular leak 410 and a relatively larger paravalvular leak 420. A paravalvular leak is gap between the outer/outermost perimeter or surface of the replacement valve (e.g., a prosthetic valve cuff) and the natural heart tissue (e.g., native annulus).

Fig. 4B is a diagrammatic top view of the replacement valve 300 of Fig. 4A in the closed position, according to aspects of the present disclosure. Visible are the heart wall 210, replacement valve wall 306, leaflets 308, small paravalvular leak 410, and larger paravalvular leak 420. In the closed position, the leaflets 308 should cause blood to stop flowing through the valve. However, because blood can flow through the paravalvular leaks 410 and 420, the functioning of the replacement valve 300 may be significantly compromised.

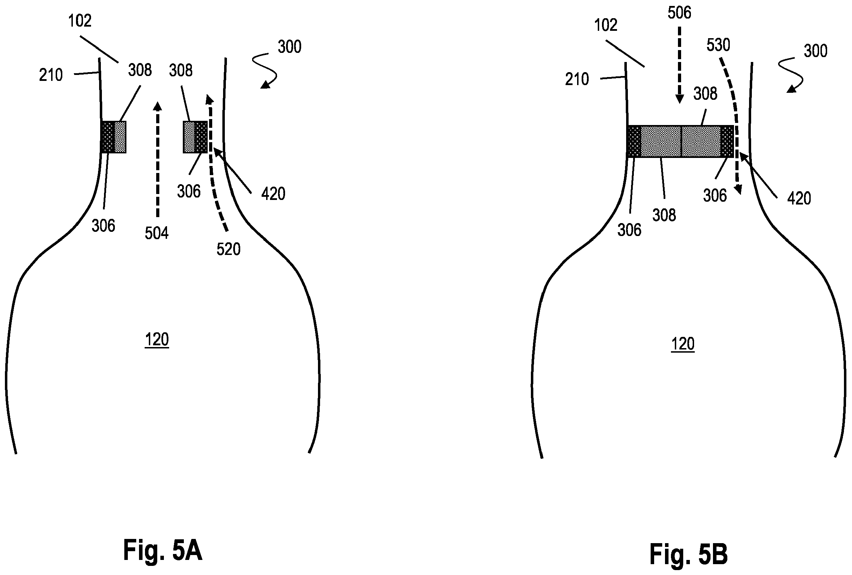

Fig. 5A is a diagrammatic cross-sectional side view of a replacement valve 300 according to aspects of the present disclosure. Visible are the natural heart wall 210 and the replacement valve leaflets 308. In the example shown in Fig. 5A, during systole (indicated by directional arrows 504), the valve leaflets 308 open and allow blood to flow from the left ventricle 120 to the aorta 102 indicated by directional arrows 504. However, paravalvular leak 420 allows leakage flow 520 to bypass the valve. Such leakage flow 520 is in the same direction as the normal blood flow 504, but may adversely impact the flow velocity, flow volume that should be entering the aorta 102.

Fig. 5B is a diagrammatic cross-sectional side views of a replacement valve 300 according to aspects of the present disclosure. Visible are the natural heart wall 210 and the replacement valve leaflets 308. In the example shown in Fig. 5B, during diastole (indicated by directional arrows 506), the valve leaflets 308 may be pushed by the flow of blood from the aorta 102 toward the left ventricle 120 such that the valve leaflets 308 close to prevent mitral regurgitation. However, the paravalvular leak 420 allows leakage flow 530 in direction 506, where blood should not be flowing at all, thus significantly compromising the function of the replacement valve 300, by effectively causing or emulating regurgitation (which may, in some cases, be a symptom for which the natural valve was replaced). Thus, to prevent such leakage flow 530, it may be critically important for the health of the patient to block or plug the paravalvular leak 420.

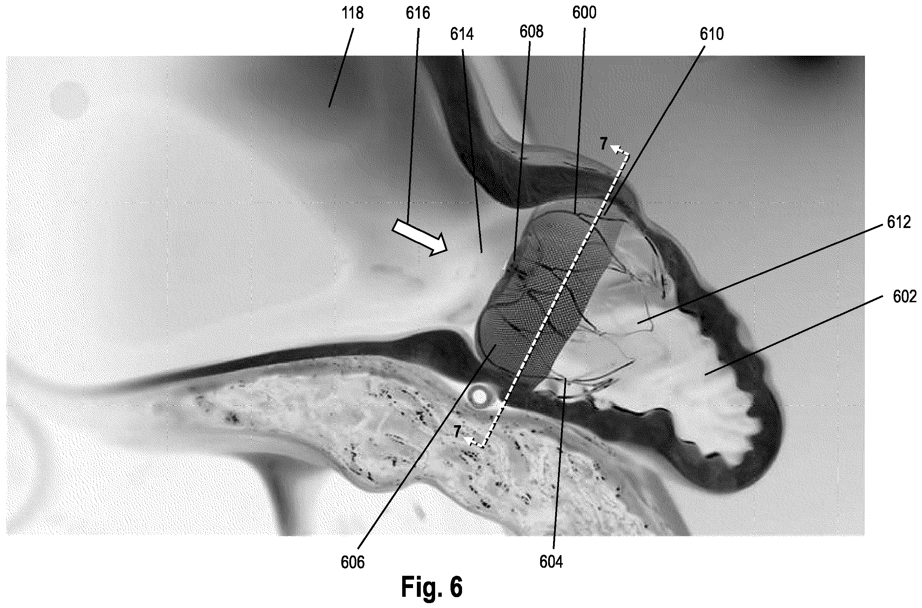

Fig. 6 is a cross-sectional view of an occlusion device 600 disposed in the left atrial appendage (LAA) of the left atrium 118 of the heart 100, according to aspects of the present disclosure. The occlusion device 600 may include a structure or cage 604 that forms an umbrella-like shape when expanded. The shape of the structure 604 may have a flat top 608 with rounded or curved sides 610 that curve around the bottom 612. The bottom 612 may be open. A mesh 606 may be disposed over and coupled to the top 608 and/or sides 610 of the structure 604. Thus, the mesh 606 may move with the structure 604 when it is expanded and/or contracted.

The occlusion device 600 may be delivered to the LAA 602 via a catheter such that the occlusion device 600 is folded within the catheter before deployment. When the distal end of the catheter is disposed proximate the opening 614 of the LAA 602, the occlusion device 600 may be deployed such that the top 608 is disposed proximate to or at the opening 614 and the bottom 612 and/or sides 610 are disposed within the LAA 602. When the occlusion device 600 exits the catheter, it may expand into the umbrella-like shape described above until the sides 610 contact the walls of the LAA 602. In some aspects, the top 608 and/or bottom 612 also contact the walls of the LAA 602. When the occlusion device 600 contacts the walls of the LAA 602, it may prevent blood flow into the LAA 602 (as shown by arrow 616), thereby minimizing the chance of blood clots developing within the LAA 602.

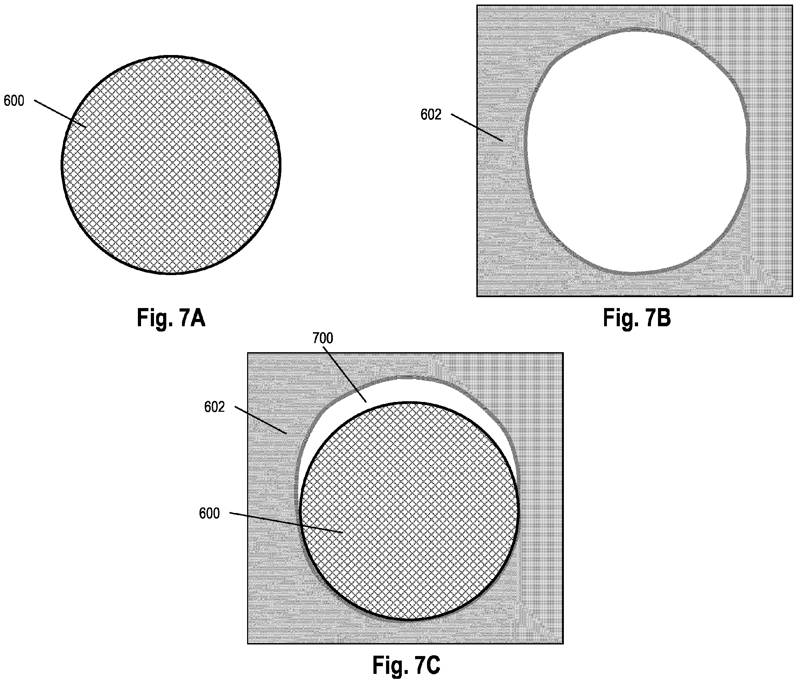

However, in some aspects, the occlusion device 600 may not completely close off the LAA 602. Figs 7A-7C illustrate an exemplary aspect in which the occlusion device 600 does not fully close off the LAA 602. Fig. 7A illustrates a diagrammatic cross-sectional view of the occlusion device 600 along the section line 7-7 in Fig. 6 from the direction indicated by the blood flow arrow 616, according to aspects of the present disclosure. This cross-section may be taken along the part of the occlusion device 600 with the largest diameter, which contacts the walls of the LAA. In the illustrated aspect, the cross-section of the occlusion device 600 is generally circular. In other aspects, the cross-section may be ovular, oblong, or any other suitable shape.

Fig. 7B illustrates a diagrammatic cross-sectional view of the walls of the LAA 602 along section line 7-7 in Fig. 6 from the direction indicated by the blood flow arrow 616, according to aspects of the present disclosure. In the illustrated aspect, the cross-section of the LAA 602 is oblong such that it is not perfectly circular. In other aspects, the cross-section may be circular, ovular, or any other suitable shape.

Fig. 7C illustrates a diagrammatic cross-sectional view of the occlusion device 600 deployed within the LAA 602 along section line 7-7 in Fig. 6 from the direction indicated by the blood flow arrow 616, according to aspects of the present disclosure. Because the cross-section of the occlusion device 600 is circular and the cross-section of the LAA 602 is non-circular, the occlusion device 600 does not continuously oppose the walls of the LAA 602. Thus, a gap 700 exists between part of the occlusion device 600 and the wall of the LAA 602. As shown in Fig. 7C, this gap 700 may be generally crescent-shaped or another non-circular shape. As described above in reference to deployment of a replacement valve 300 in Figs 5A-5B, blood flow may move through the gap between the device and the native tissue. Thus, when a gap 700 exists between the wall of the LAA 602 and the occlusion device 600, blood flow may enter and exit the LAA 602 via the gap 700. This may prevent the occlusion device 600 from fully sealing the LAA 602 as desired and may allow blood clots to form within the LAA 602.

Thus, when there are gaps (e.g. gaps 410, 420, 700) formed between the anatomy (e.g. heart wall 210 or LAA wall 602) and a therapeutic device (e.g. a replacement valve 300 or an occlusion device 600), this may prevent the therapeutic device from functioning as desired or needed. Thus, it may be advantageous to plug or close these gaps to prevent unwanted blood flow around the therapeutic device.

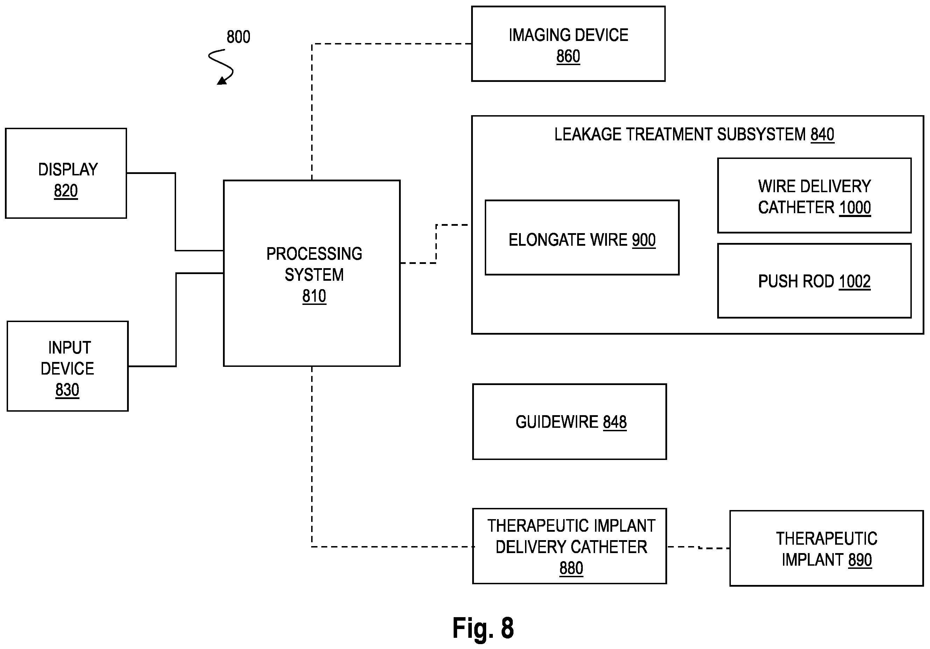

Fig. 8 is schematic, diagrammatic view of a system 800 that may prevent leakage between a therapeutic device or implant 890 and the anatomy of the patient, according to aspects of the present disclosure. The system 800 may be configured to evaluate (e.g., assess), display, and/or control (e.g., modify) one or more aspects of the delivery and/or deployment of a therapeutic implant 890 or the delivery and/or deployment of an implantable elongate wire 900. For instance, the system 800 may be utilized to monitor and/or control one or more portions of the delivery and/or deployment of a therapeutic implant 890 or the delivery and/or deployment of an elongate wire 900. In this regard, the system 800 may be used to assess coronary vessels and/or heart tissue (e.g., the myocardium). As illustrated, the system 800 may include a processing system 810 in communication with a display device 820 (e.g., an electronic display or monitor), an input device 830 (e.g., a user input device, such as a keyboard, mouse, joystick, microphone, and/or other controller or input device), a leakage treatment subsystem 840 and/or an imaging device 860 (e.g., x-ray or radiographic imaging, fluoroscopy, computed tomography or CT, magnetic resonance imaging or MRI, etc.). As illustrated, the system 800 may further include a therapeutic implant delivery catheter 880 and a therapeutic implant 890. As explained above, the therapeutic implant 890 may be a replacement valve 300 (e.g. a prosthetic, mechanical, or donor valve) or an occlusion device 600 (e.g. for closing off the LAA 602). In some aspects, delivery and/or deployment of the therapeutic implant 890 via the therapeutic implant delivery catheter 880 may be completely mechanical (no connection to the processing system 810) or may be connected to processing system 810 (e.g., for control of movement and/or deployment of therapeutic implant). It is understood that other types of therapeutic implants and therapeutic implant delivery systems may be used instead of or in addition to those described herein.

The leakage treatment subsystem 840 includes a wire delivery catheter 1000, a push rod 1002, and an elongate wire 900 that can be deployed from the wire delivery catheter 1000 by being pushed out by the push rod 1002, as described in more detail below. In some aspects, delivery and/or deployment of the elongate wire 900 via the wire delivery catheter 1000 may be completely mechanical (no connection to the processing system 810) or may be connected to processing system 810 (e.g., for control of movement and/or deployment of the elongate wire 900).

Either or both of the wire delivery catheter 1000 and the therapeutic implant delivery catheter 880 may be guided over a guidewire 848.

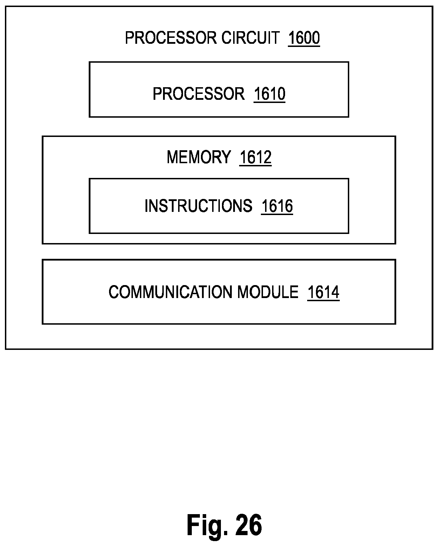

The processing system 810 is generally representative of any device suitable for performing the processing and analysis techniques disclosed herein. In some aspects, the processing system 810 includes a processor circuit, such as the processor circuit 1600 of Fig. 26, which is described in more detail below. In some aspects, the processing system 810 is programmed to execute steps associated with the data acquisition, analysis, and/or instrument (e.g., device) control described herein. Accordingly, it is understood that any steps related to data acquisition, data processing, instrument control, and/or other processing or control aspects of the present disclosure may be implemented by the processing system 810 (e.g., computing device) using corresponding instructions stored on or in a non-transitory computer readable medium accessible by the computing device. In some instances, the processing system 810 is a console device. Further, it is understood that in some instances the processing system 810 includes one or a plurality of computing devices, such as computers, with one or a plurality of processor circuits. In this regard, it is particularly understood that the different processing and/or control aspects of the present disclosure may be implemented separately or within predefined groupings using a plurality of computing devices. Any divisions and/or combinations of the processing and/or control aspects described below across multiple computing devices are within the scope of the present disclosure.

The system 800 is configured such that when the wire delivery catheter 1000 is positioned within the heart, images captured by the imaging system 860 can show the location and orientation of the wire delivery catheter 1000, and also potentially anatomical features such as the therapeutic implant 890, heart wall, and heart valve leaflets.

It is noted that block diagrams are provided herein for exemplary purposes; a person of ordinary skill in the art will recognize myriad variations that nonetheless fall within the scope of the present disclosure. For example, block diagrams may show a particular arrangement of components, subcomponents, modules, units, etc. It is understood that some aspects of the systems disclosed herein may include additional components, that some components shown may be absent from some aspects, and that the arrangement of components may be different than shown, while still performing the methods described herein.

It is understood that, in some instances, one or more components of the system 800 can operate without one or more other components of the system 800. For example, the leakage treatment subsystem 840, guidewire 848, therapeutic implant delivery catheter 880, and/or therapeutic implant 890 can be implemented without the processing system 810. For example, the leakage treatment subsystem 840, guidewire 848, therapeutic implant delivery catheter 880, and/or therapeutic implant 890 can be mechanical components that do not have signal communication with the processing system 810.

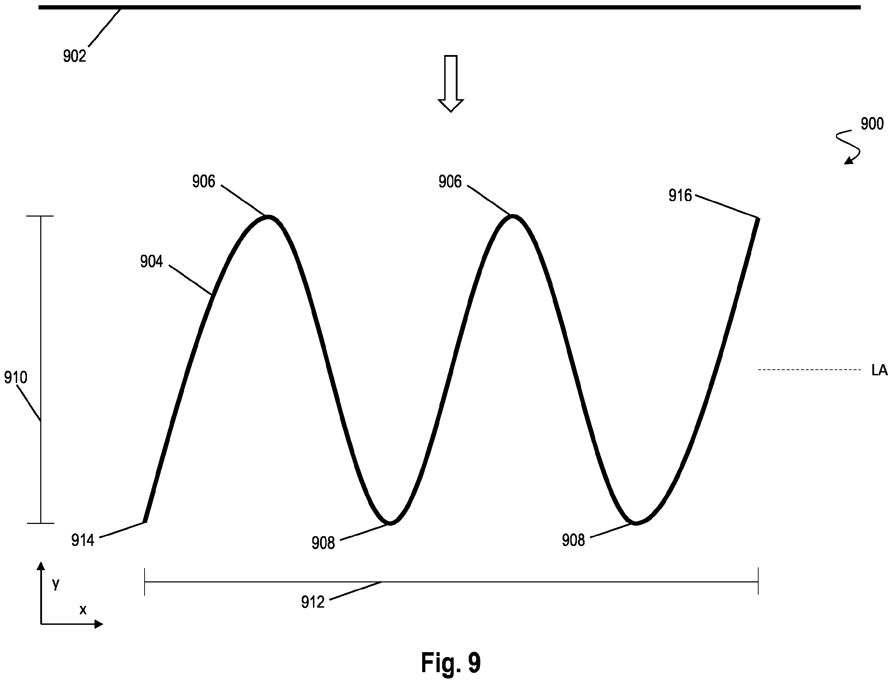

Fig. 9 illustrates a diagrammatic front view of an elongate wire 900, according to some aspects. For example, the front view may illustrate the elongate wire 900 along an x-y plane such that the x-dimension (i.e. lateral dimension) is oriented in the horizontal direction and the y-dimension (i.e. longitudinal dimension) is oriented in the vertical direction. The elongate wire 900 may be formed of a single piece of straight wire 902, as shown at the top of Fig. 9. The straight wire 902 is then set, deformed, or bent into a serpentine shape 904, as shown at the bottom of Fig. 9. The straight wire 902 may be deformed by any suitable process. For example, the straight wire 902 may be heat set into the serpentine shape 904. In some aspects, the wire 900 may be bent into the serpentine shape 904 before it is inserted into a wire delivery catheter 1000. In other aspects, the wire 900 may be bent into the serpentine shape 904 during or after it exits the wire delivery catheter 1000. For example, the delivery catheter 1000 could include rollers on opposite sides that a straight wire passes through, causing the wire to change shape into the serpentine/sinusoidal shape.

The serpentine shape 904 may be described as a sinusoidal shape having a plurality of peaks 906 and a plurality of troughs 908 longitudinally spaced from the plurality of peaks 906 along the y-dimension. The peaks 906 may also be laterally offset from the troughs 908 along the x-dimension. Each peak 906 may be laterally offset from the neighboring peaks 906 along the x-dimension. Similarly, each trough 908 may be laterally offset from the neighboring troughs 908 along the x-dimension. Thus, the serpentine shape 904 may have a sinusoidal or wave-like shape along the x-y plane that curves between the peaks 906 and troughs 908 between a first end 914 and second end 916 of the wire 900. The longitudinal axis (LA) of the elongate wire 900 may be parallel to the y-dimension and equidistant between the peaks 906 and troughs 908.

The serpentine shape 904 of the elongate wire 900 may allow the elongate wire 900 to be resilient and/or flexible in the x-dimension and/or y-dimension. In some aspects, the elongate wire 900 is flexible and resilient such that the serpentine shape 904 can be non-destructively deformed (e.g., flexed) and the elongate wire 900 will resiliently return to its original shape. As explained in more detail below, this may allow the elongate wire 900 to be compressed or constrained into the wire delivery catheter 1000 and/or the gap between the therapeutic implant 890 and the neighboring tissue. In some aspect, the serpentine shape 904 may allow the wire 900 to function like a compression spring that is biased outward.

The elongate wire 900 may have any suitable dimensions. For example, the serpentine shape 904 may have an amplitude 910 (i.e. a distance between the peaks 906 and troughs 908) of approximately 0.1 millimeters (mm), 0.2 mm, 0.3 mm, 0.4 mm, 0.5 mm, 0.6 mm, 0.7 mm, 0.8 mm, 0.9 mm, 1 mm, 1.5 mm, 2 mm, 2.5 mm, 3 mm, 4 mm, 5 mm, 6 mm, 7 mm, 8 mm, 9 mm, 10 mm, or any value therebetween.

Two peaks 906 and two troughs 908 are illustrated in Fig. 9, though one peak 906 or three or more peaks 906 and one trough 908 or three or more troughs 908 are contemplated. The serpentine shape 904 can have any suitable number of peaks 906 and troughs 908 (e.g., one or more multiple peaks 906, and one or multiple troughs 908), depending on the length 912 of the elongate wire 900 and/or the frequency/wavelength of the serpentine/sinusoidal shape 904. For example, a longer total length 912 can lead to a greater number of peaks/troughs 906, 908 and a short total length 912 can lead to a lesser number of peaks/troughs 906, 908. A greater frequency (or shorter wavelength) can lead to a greater number of peaks/troughs 906, 908, and a lesser frequency (or longer wavelength) can lead to a lesser number of peaks/troughs 906, 908. An example wavelength can be 0.1 mm, 0.2 mm, 0.3 mm, 0.4 mm, 0.5 mm, 0.6 mm, 0.7 mm, 0.8 mm, 0.9 mm, 1 mm, 1.5 mm, 2 mm, 2.5 mm, 3 mm, 4 mm, 5 mm, 6 mm, 7 mm, 8 mm, 9 mm, 10 mm, or any value therebetween.

Moreover, the serpentine shape 904 may have length 912 (i.e. a distance between the first end 914 and the second end 916 of approximately 0.1 mm, 0.2 mm, 0.3 mm, 0.4 mm, 0.5 mm, 0.6 mm, 0.7 mm, 0.8 mm, 0.9 mm, 1 mm, 1.5 mm, 2 mm, 2.5 mm, 3 mm, 4 mm, or any value therebetween.

In some aspects, the full length 912 of the elongate wire 900 may be inserted into the gap. In other aspects, the elongate wire 900 may be cut so that only a portion of the length 912 is inserted into the gap. Thus, the elongate wire 900 may have a relatively long length 912 and may be cut to the desired length during deployment as described in more detail below.

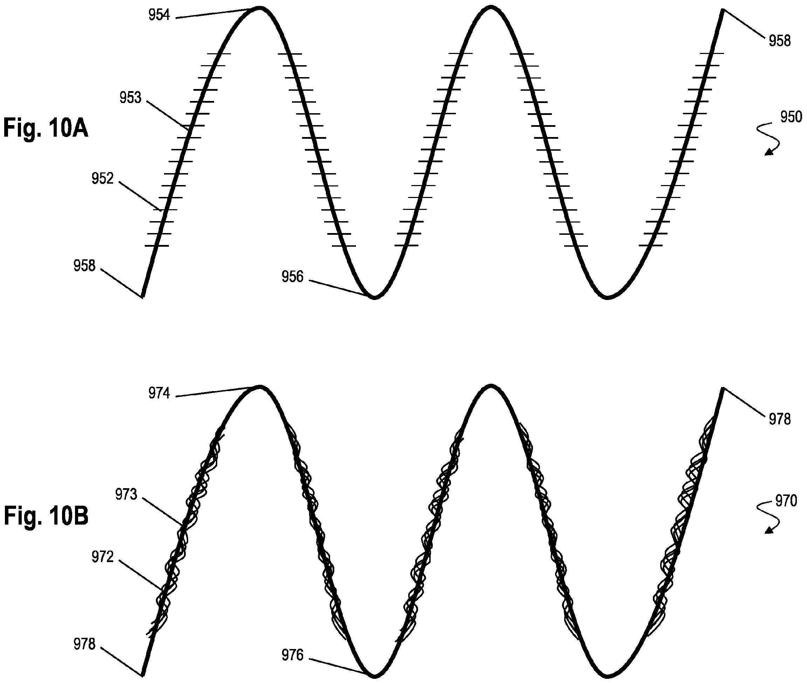

Figs 10A-10B illustrate elongate wires that promote endothelialization and/or attachment or engagement with the tissue and/or therapeutic implant 890. Fig. 10A illustrates an elongate wire 950 having a plurality of barbs 952 disposed thereon, according to some aspects of the present disclosure. In some aspects, the barbs 952 may be disposed along the sloped portion 953 of the wire between the peaks 954 and troughs 956. The barbs 952 may not be disposed on the ends 958 of the elongate wire 950. In other aspects, the barbs 952 may be disposed on the peaks 954, troughs 956, and/or ends 958. The barbs 952 may be formed of the single wire or may be separate parts coupled and/or attached to the single wire.

Fig. 10B illustrates an elongate wire 970 having a plurality of fibers 972 disposed thereon, according to some aspects of the present disclosure. In some aspects, the fibers 972 may be disposed along the sloped portion 973 of the wire between the peaks 974 and troughs 976. The fibers 972 may not be disposed on the ends 978 of the elongate wire 970. In other aspects, the fibers 972 may be disposed on the peaks 974, troughs 976, and/or ends 978. The fibers 972 may be formed of the single wire or may be separate parts coupled and/or attached to the single wire.

The barbs 952 or fibers 972 may increase the contact area of the elongate wire 950, 970 so that the wire 950, 970 has increased contact with the therapeutic implant 890 and/or tissue. Thus, the barbs 952 and/or fibers 972 may improve engagement or attachment to the therapeutic implant 890 and/or tissue by preventing movement of the elongate wire 950, 970 once it is implanted. Moreover, the barbs 952 and fibers 972 may increase endothelialization around the elongate wire 950, 970, further closing and/or filling the gap in which the wire 950, 970 is implanted. Any suitable part that increases engagement or attachment to the therapeutic implant 890 and/or tissue or promotes endothelialization may be used in place of barbs 952 or fibers 972.

The elongate wire 900 may be formed of any suitable material. For example, the wire 900 may be formed of a metal or metal alloy such as stainless steel, nickel, titanium, nitinol, cobalt, chromium, any alloy thereof, or any other suitable metal. In other aspects, the wire 900 may be formed of a polymer such as ultra-high molecular weight polyethylene, polyether ether ketone, shape memory polymers, or any other suitable polymer. The barbs 952 may be formed of any of the metal, metal alloys, or polymers listed above. In some aspects, the barbs 952 may be formed of an absorbable material such as an absorbable sutures including, for example, polydioxanone, polyglycolic acid, polyglyconate, polylactic acid, collagen (which may be derived from an animal), or any other suitable material. Similarly, the fibers 972 may be formed of any suitable material, including for example, any of the metal, metal alloys, or polymers listed above. The fibers 972 may also be formed of an absorbable material such as an absorbable sutures including, for example, polydioxanone, polyglycolic acid, polyglyconate, polylactic acid, collagen (which may be derived from an animal), or any other suitable material.

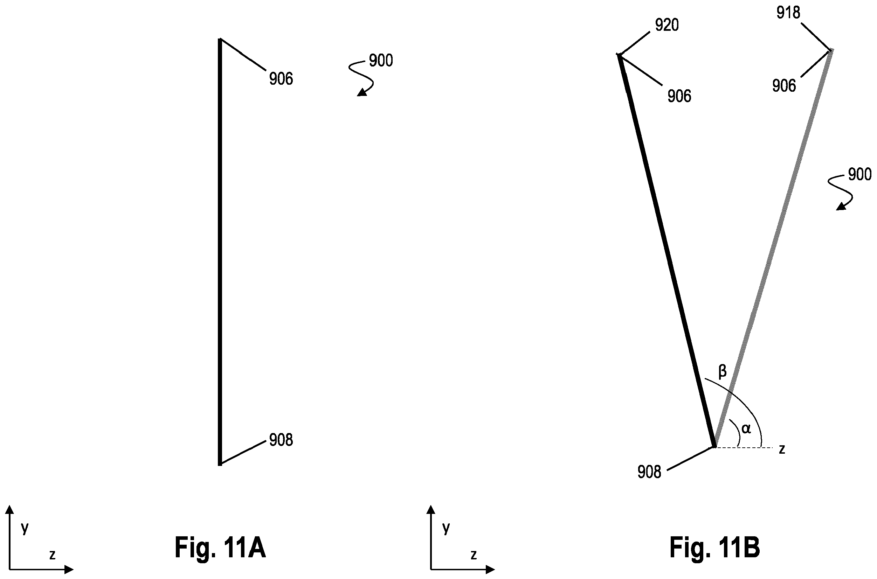

Fig. 11A illustrates a side view of the elongate wire 900 shown in Fig. 9 in which the peaks 906 and troughs 908 are aligned, according to aspects of the present disclosure. The side view may illustrate the elongate wire 900 along an y-z plane such that the z-dimension (i.e. depth or thickness dimension) is oriented in the horizontal direction and the y-dimension is oriented in the vertical direction. In some aspects, the peaks 906 and troughs 908 are aligned in the z-dimension. Thus, the elongate wire 900 only extends in the y-dimension (and x-dimension as shown in Fig. 9) and does not extend in the z-dimension, thereby forming a single vertical line in the y-z plane.

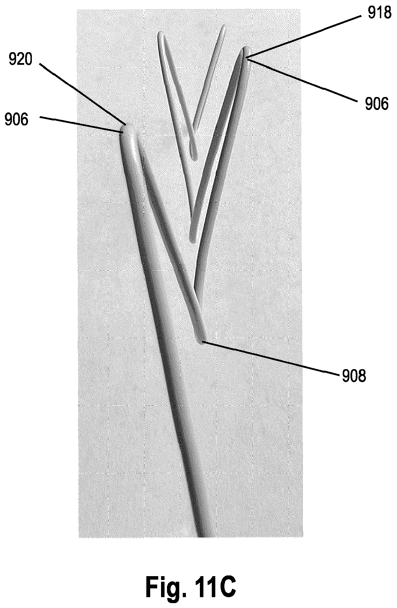

Figs 11B-11C illustrate diagrammatic views of the elongate wire 900 shown in Fig. 9 in which the peaks 906 and troughs 908 are not aligned, according to aspects of the present disclosure. Fig. 11B illustrates a side view of the elongate wire 900. As in Fig. 11A, the side view may illustrate the elongate wire 900 along an y-z plane such that the z-dimension (i.e. depth or thickness dimension) is oriented in the horizontal direction and the y-dimension is oriented in the vertical direction. Fig. 11C illustrates a perspective side view of the elongate wire 900.

In some aspects, a peak 906 may be spaced from (i.e. may not be aligned with) neighboring peaks 906 along the z-dimension. The troughs 908 may be not be spaced from (i.e. may be aligned with) neighboring troughs 908 in the z-dimension. Thus, the side view of the elongate wire 900 may be a V-shape, as shown in Fig. 11B. A first set 918 of peaks 906 may be angled relative to the z-dimension at an angle α. Angle α may be any suitable value. For example, angle α may be any value between 0 degrees and 90 degrees. Additionally, a second set 920 of peaks 906 may be angled relative to the z-dimension at an angle β. Angle α may be any suitable value. For example, angle β may be any value between 90 degrees and 180 degrees. Angle α may or may not be the same as angle β.

Therefore, the serpentine shape 904 may be three-dimensional (3D) such that it extends in the x, y, and z-dimensions. When the serpentine shape 904 is 3D, this may allow the wire 900 to better contact and/or be maintained within the gap. The 3D shape may engage the therapeutic implant 890 and/or tissue so that the engagement prevents the wire 900 from falling or moving out of the gap.

In other aspects, the troughs 908 may not be aligned with neighboring troughs 908 in the z-dimension and the peaks 906 may be aligned with neighboring peaks 906 in the z-dimension. Thus, the side view may be a V-shape (an inverted version of the side view shown in Fig. 11B). In yet other aspects, both the peaks 906 and troughs 908 may be misaligned from neighboring peaks 906 and troughs 908, respectively. In some embodiments, each trough 908 and/or 906 peak may be misaligned from every other trough 908 and/or peak 906.

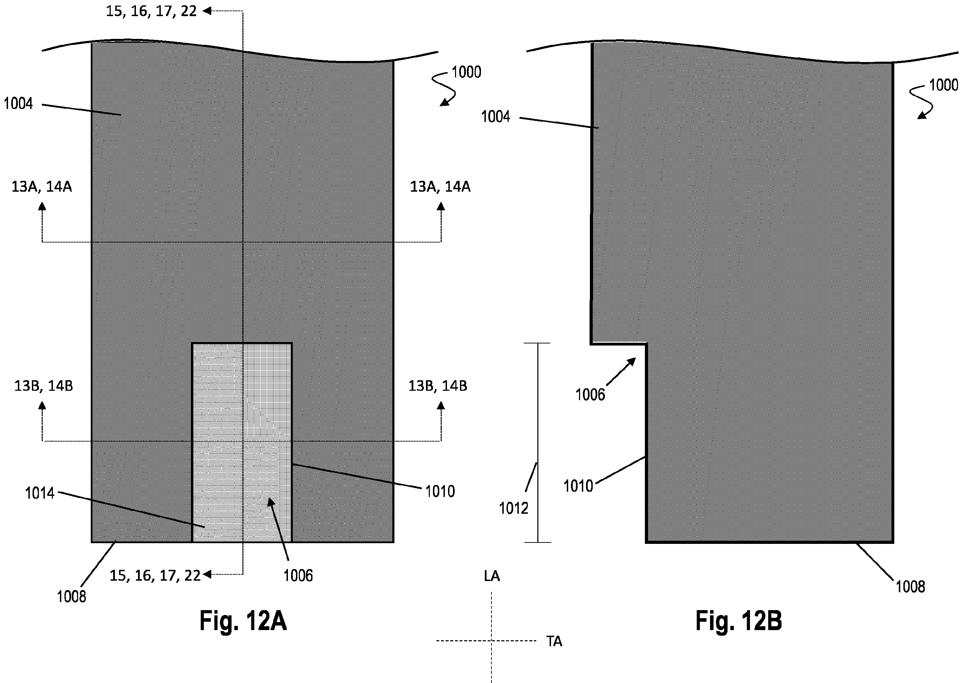

The elongate wire 900 may be delivered to the site of the therapeutic implant 890 and deployed, inserted, or implanted into a gap using a wire delivery catheter 1000 and a push rod 1002. Figs 12A-12B illustrate a wire delivery catheter 1000, according to some aspects of the present disclosure. Fig. 12A illustrates a diagrammatic front view of the wire delivery catheter 1000 and Fig. 12B illustrates a diagrammatic side view of the wire delivery catheter 1000. The wire delivery catheter 1000 may include a tube 1004 having a lumen 1006 extending from a proximal end to a distal end 1008 of the tube 1004. An inner surface 1014 of the catheter 1000 may define the lumen 1006. The catheter 1000 may have a longitudinal axis (LA) and a transverse axis (TA) perpendicular to the LA.

A longitudinal slit, slot, opening, or notch 1010 may extend upward from the distal end 1008 of the catheter 1000. The notch 1010 may extend from the outer surface of the catheter 1000 to the inner surface 1014 so that the notch 1010 is in communication with the lumen 1006. The elongate wire 900 may exit the catheter 1000 through the notch 1010. Thus, the notch 1010 may be configured to allow the elongate wire 900 to exit at an angle to the LA of the catheter 1000. For example, the elongate wire 900 may exit perpendicularly to the LA (i.e. parallel to the TA) or may exit at an angle between 0 degrees to 90 degrees relative to the LA.

The notch 1010 may have a length 1012. The length 1012 may be any suitable value. For example, the length 1012 may be approximately 0.1 mm, 0.2 mm, 0.3 mm, 0.4 mm, 0.5 mm, 0.6 mm, 0.7 mm, 0.8 mm, 0.9 mm, 1 mm, 1.5 mm, 2 mm, 2.5 mm, 3 mm, 4 mm, 5 mm, 6 mm, 7 mm, 8 mm, 9 mm, 10 mm, or any value therebetween.

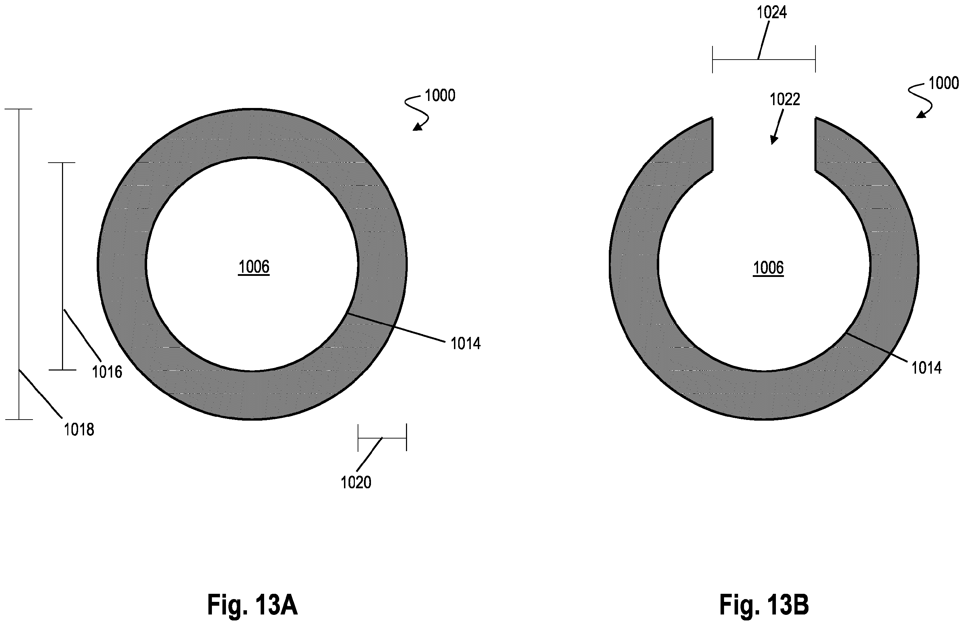

Figs 13A-13B illustrate diagrammatic cross-sectional views of the catheter 1000 illustrated in Figs 12A-12B. Fig. 13A illustrates a cross-sectional view through the 13A-13A line in Fig. 12A, according to some aspects of the present disclosure. In some aspects, the catheter 1000 may have a circular cross-section. Thus, the catheter 1000 may be generally cylindrical.

The catheter 1000 may have an inner diameter 1016, an outer diameter 1018, and a thickness 1020 (i.e. a distance between the inner diameter 1016 and the outer diameter 1018). The inner diameter 1016 may be any suitable value. For example, the inner diameter 1016 may be approximately 0.1 mm, 0.2 mm, 0.3 mm, 0.4 mm, 0.5 mm, 0.6 mm, 0.7 mm, 0.8 mm, 0.9 mm, 1 mm, 1.5 mm, 2 mm, 2.5 mm, or any value therebetween. The inner diameter 1016 may be large enough to prevent plastic deformation of the wire 900, but small enough to prevent the wire 900 from bunching and/or tangling as it exits the catheter 1000.

The outer diameter 1018 may be any suitable value. For example, the outer diameter 1018 may be approximately 0.1 mm, 0.2 mm, 0.3 mm, 0.4 mm, 0.5 mm, 0.6 mm, 0.7 mm, 0.8 mm, 0.9 mm, 1 mm, 1.5 mm, 2 mm, 2.5 mm, or 3 mm. The thickness 1020 may be any suitable value. For example, the thickness 1020 may be approximately 0.1 mm, 0.2 mm, 0.3 mm, 0.4 mm, 0.5 mm, 0.6 mm, 0.7 mm, 0.8 mm, 0.9 mm, 1 mm, 1.5 mm, 2 mm, or any value therebetween.

Fig. 13B illustrates a cross-sectional view through the 13B-13B line in Fig. 12A, according to some aspects of the present disclosure. Thus, Fig. 13B illustrates a cross-sectional view of the catheter 1000 through the notch 1010. The notch 1010 may have a width 1024. The width 1024 may be any suitable value. For example, the width 1024 may be approximately 0.1 mm, 0.2 mm, 0.3 mm, 0.4 mm, 0.5 mm, 0.6 mm, 0.7 mm, 0.8 mm, 0.9 mm, 1 mm, 1.5 mm, 2 mm, 2.5 mm, 3 mm, or any value therebetween. In some aspects, the width 1024 of the notch 1010 may be slightly larger than the diameter of the wire 900 so that the notch 1010 is just wide enough so that the wire 900 can exit therethrough.

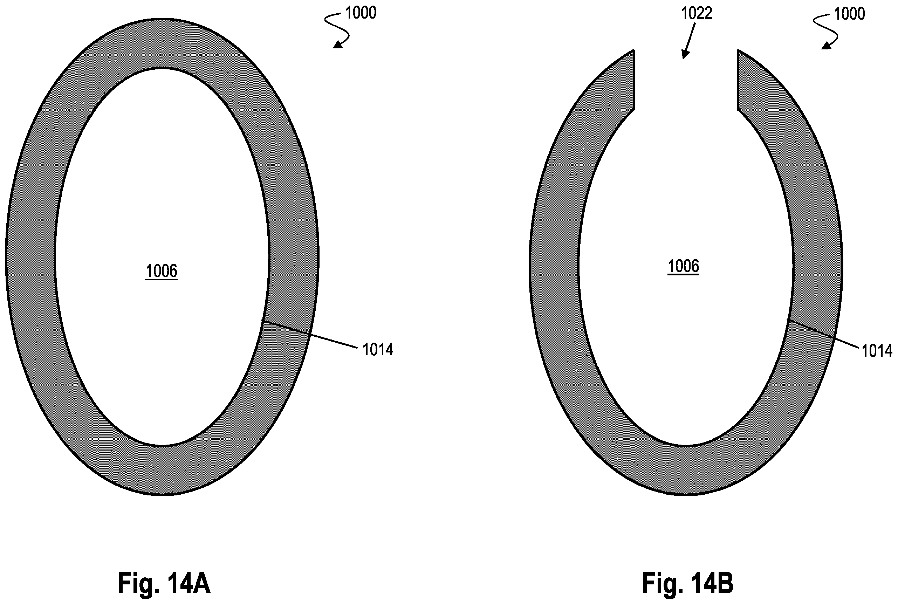

Figs 14A-14B illustrate another example of diagrammatic cross-sectional views of the catheter 1000. Fig. 14A illustrates a cross-sectional view through the 14A-14A line in Fig. 12A (which is the same as the 13A-13A line), according to some aspects of the present disclosure. Fig. 14B illustrates a cross-sectional view through the 14B-14B line in Fig. 12A (which is the same as the 13B-13B line), according to some aspects of the present disclosure. Thus, Fig. 14B illustrates a cross-sectional view of the catheter 1000 through the notch 1010. In some aspects, the cross-section of the catheter 1000 may not be circular. As shown in Figs 14A-14B, the cross-section may be ovular. In some aspects, the notch 1010 may be located along a more narrow end of the ovular cross-section, as shown in Fig. 14B. In other aspects, the notch 1010 may be disposed on a wider side of the ovular cross-section. In some aspects, the cross-section may be any suitable shape. For example, the cross-section may be rectangular, octagonal, or any other suitable shape. In some aspects, the shape of the cross-section may be different at different points along the length of the catheter 1000.

When the cross-section of the catheter 1000 is oval-shaped, the notch 1010 may be located on the more narrow end of the oval so that the orientation of the notch 1010 is easier to determine. For example, when imaging the catheter 1000 within the body (e.g. via radiographic or fluoroscopic imaging), it may be difficult to determine the orientation of the catheter 1000 and, thus, the orientation of the notch 1010. By having a catheter 1000 with an ovular cross-section, the physician can determine the orientation of the notch 1010 by determining the location of the narrow end of the oval-shape. Moreover, the oval-shape may better orient the wire 900 so that it can more easily exit the catheter 1000 through the notch 1010.

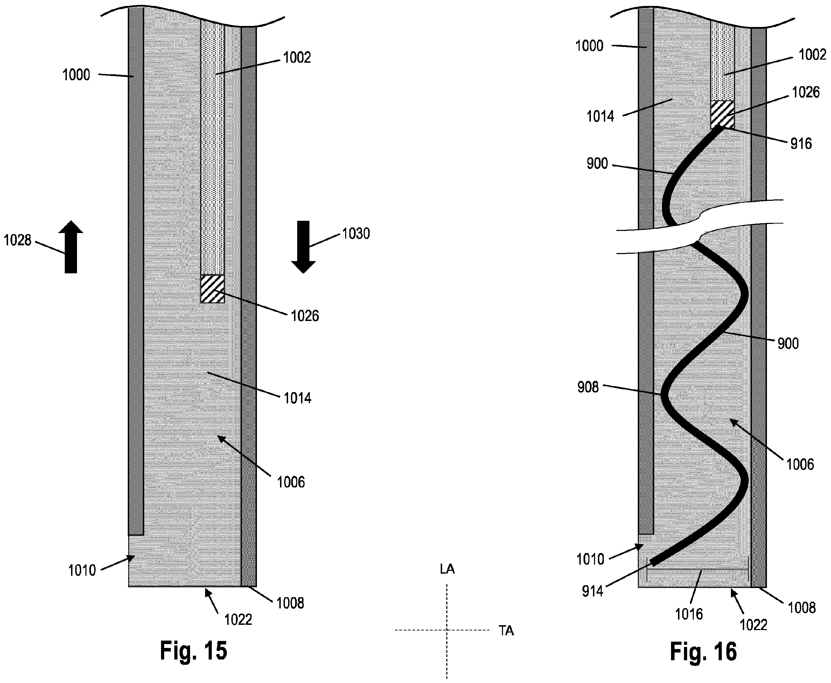

Fig. 15 illustrates a diagrammatic cross-sectional view of the catheter 1000 through the 15-15 line in Fig. 12A with the push rod 1002 disposed therein, according to some aspects of the present disclosure. The push rod 1002 may be disposed within the lumen 1006 of the catheter 1000. The push rod 1002 may be elongate and may extend from a proximal end (not shown) to a distal end 1026. The distal end 1026 of the push rod 1002 may be configured to contact and press against the second end 916 of the elongate wire 900. In some aspects, the distal end 1026 may include a coupling element that removably couples or attaches to the second end 916 of the elongate wire 900. For example, the coupling element may be a loop or a hook. In other examples, the coupling element may have a male/female thread and the second end 916 of the wire 900 may have the other of a male/female thread, such that the two can be threadedly coupled. In yet other examples, the coupling element may have an adhesive or Velcro-like element that removably couples to the second end 916 of the wire 900.

In some aspects, the distal end 1026 of the push rod 1002 may not have a coupling element and may instead have a bottom surface that is configured to contact and push against the second end 916 of the wire 900 without coupling or attaching thereto. This may advantageously allow the wire 900 to be pushed out of the catheter 1000 without a separate step of uncoupling or detaching a coupling element of the push rod 1002 from the wire 900. Moreover, this may allow multiple wires 900 or a portion of a longer wire 900 to be deployed without removing the catheter 1000 and/or actuator from the body.

The push rod 1002 may be moveable proximally (as shown by arrow 1028) or distally (as shown by arrow 1030) within the lumen 1006 along the LA of the catheter 1000.

The distal end of the catheter 1000 may include a distal opening 1022. In some aspects, the elongate wire 900 may exit the catheter 1000 through the notch 1010 and/or the distal opening 1022. In some aspects, the distal end 1008 of the catheter 1000 may be closed off or include a bottom. Thus, the elongate wire 900 may only exit through the notch 1010.

Fig. 16 illustrates a diagrammatic cross-sectional view of the catheter 1000 through the 16-16 line in Fig. 12A (which is the same as the 15-15 line) with the push rod 1002 and elongate wire 900 disposed therein, according to some aspects of the present disclosure.

The elongate wire 900 may be disposed within the lumen 1006 of the catheter 1000 such that the first end 914 points towards the proximal end (not shown) of the catheter 1000 and the second end 916 points to the distal end of the catheter 1000. The second end 916 of the wire 900 may be disposed within or proximate to the notch 1010 of the catheter 1000. In some aspects, the LA of the wire 900 may be aligned with the LA of the catheter 1000.

In some aspects, the inner diameter 1016 of the catheter 1000 may be smaller than the amplitude 910 of the heat-set serpentine shape 904 of the elongate wire 900. Thus, the peaks 906 of the serpentine shape 904 may contact one side of the inner surface 1014 of the catheter 1000 and the troughs 908 may contact the opposite side of the inner surface 1014. Thus, the inner surface 1014 of the catheter 1000 may compress or constrain the serpentine shape 904 of the wire 900 such that the amplitude is decreased relative to its heat-set shape and is approximately the same as the inner diameter 1016 of the catheter 1000. In some aspects, the inner diameter 1016 of the catheter 1000 may be relatively small such that it constrains the wire 900 to form a generally straight shape within the lumen 1006. Thus, in some aspects, when the inner surface 1014 constrains the wire 900, the wire 900 may become more straight and less curved or sinusoidal. In some aspects, the distance between the peaks 906 and troughs 908 of the serpentine or sinusoidal shape 904 (which may be comparable to a wavelength measurement) may be larger when constrained within the lumen 1006 of the catheter 1000 as compared to that of the original heat-set serpentine shape 904.

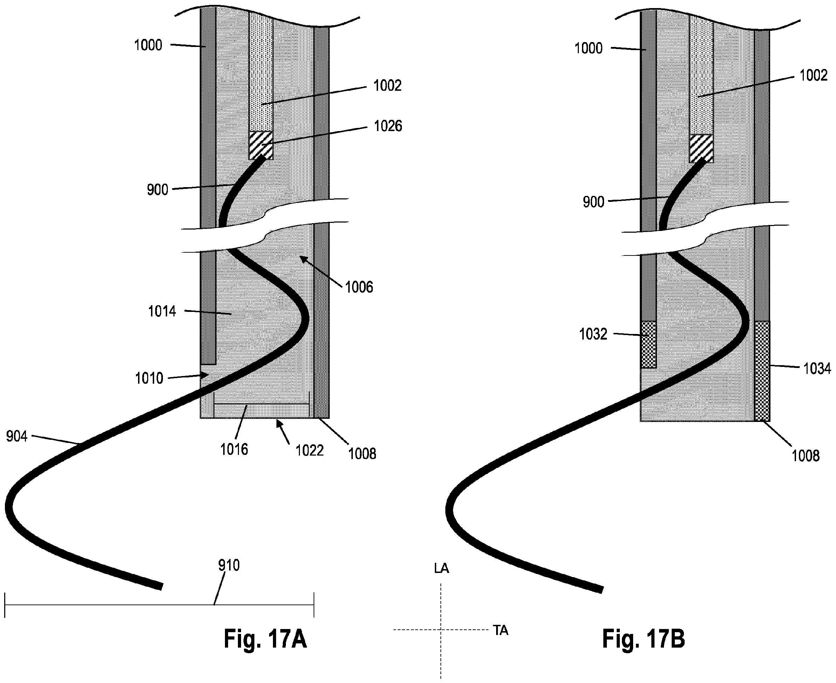

Figs 17A illustrates a diagrammatic cross-sectional view of the catheter 1000 through the 17-17 line in Fig. 12A (which is the same as the 15-15 and 16-16 lines) with the push rod 1002 moving the elongate wire 900 out of the catheter 1000, according to some aspects of the present disclosure. The push rod 1002 may push the wire 900 out of the distal end 1008 of the catheter 1000 via the notch 1010. The distal end 1026 of the push rod 1002 may be moved distally within the lumen 1006 of the catheter 1000 to press against the second end 916 of the wire 900. This may move the wire 900 distally within the lumen 1006 and out of the notch 1010.

The notch 1010 in the catheter 1000 may allow the elongate wire 900 to exit the catheter 1000 at an angle relative to the LA of the catheter 1000. For example, the serpentine shape 904 of the elongate wire 900 may be oriented within the lumen 1006 of the catheter 1000 such that it exits the catheter generally perpendicularly to the LA of the catheter 1000 (i.e. along the TA of the catheter 1000). In some aspects, the elongate wire 900 may exit the catheter 1000 through the notch 1010 at an angle between 0 degrees to 90 degrees (or an oblique angle) relative to the LA of the catheter 1000. By deploying the wire 900 perpendicularly or at an oblique angle relative to the LA of the catheter 1000, the catheter 1000 may be moved to the gap between the therapeutic implant 890 and the tissue such that the distal end 1008 of the catheter 1000 faces the gap. The wire 900 may be deployed parallel to the gap so that the wire 900 can be better inserted therein, as described in more detail below.

As the wire 900 exits the catheter 1000, the inner surface 1014 no longer constrains the amplitude 910 of the wire 900 to the inner diameter 1016 of the catheter 1000. The original amplitude 910 of the heat-set serpentine shape 904 may be larger than the inner diameter 1016. Thus, the portion of the wire 900 outside of the catheter 1000 may resiliently return to its heat-set serpentine shape 904 at the original amplitude 910.

Fig. 17B illustrates the same view of the catheter 1000, push rod 1002, and elongate wire 900 as shown in Fig. 17B, but further including radiopaque markers 1032, 1034, according to some aspects of the present disclosure. A smaller radiopaque marker 1032 may be disposed on or within the wall of the catheter 1000 proximal to the notch 1010. The smaller radiopaque marker 1032 may extend proximally from the proximal end of the notch 1010. A larger radiopaque marker 1034 may be disposed on or within the wall of the catheter 1000 opposite the small radiopaque marker 1032 and the notch 1010. The larger radiopaque marker 1034 may extend proximally from the distal end 1008 of the catheter 1000.

The radiopaque markers 1032, 1034 may be used to determine the orientation of the catheter 1000 and, thus, the orientation of the notch 1010. During delivery and deployment of the elongate wire 900 into a gap between a therapeutic implant 890 and the tissue, an imaging device 860 such as a radiographic or fluoroscopic imaging may be used to view the catheter 1000 as it moves through a lumen of the body (e.g. through the heart). The radiopaque markers 1032, 1034 may be visible within the image of the body so that the physician can see the location of the catheter 1000.

The smaller radiopaque marker 1032 may indicate the location and orientation of the notch 1010. For example, if the image shows the smaller radiopaque marker 1032 on the left and the larger radiopaque marker 1034 on the right, this indicates that the notch 1010 is oriented or facing left. Similarly, if the image shows the smaller radiopaque marker 1032 on the right and the larger radiopaque marker 1034 on the left, this indicates that the notch 1010 is oriented or facing right. If only one radiopaque marker is visible in the radiopaque image, this may indicate that the smaller and larger radiopaque markers 1032, 1034 are aligned and the notch 1010 is oriented or facing either out of the page or into the page. In some aspects, when the smaller and larger radiopaque markers 1032, 1034 are aligned, the physician may be able to distinguish which radiopaque marker 1032, 1034 is oriented or facing into and/or out of the page. Thus, in some aspects, the physician may be able to determine whether the notch 1010 is oriented or facing into or out of the page.

In other aspects, any arrangement or combination of radiopaque markers may be used to allow the physician to determine the orientation of the catheter 1000. For example, two radiopaque markers of equal size may be disposed on or within the wall of the catheter 1000 on opposite sides of the catheter 1000. A first radiopaque marker may be proximally offset from a second radiopaque marker to indicate that the notch 1010 is located on the side of the first radiopaque marker. However, any other arrangement of radiopaque markers is contemplated.

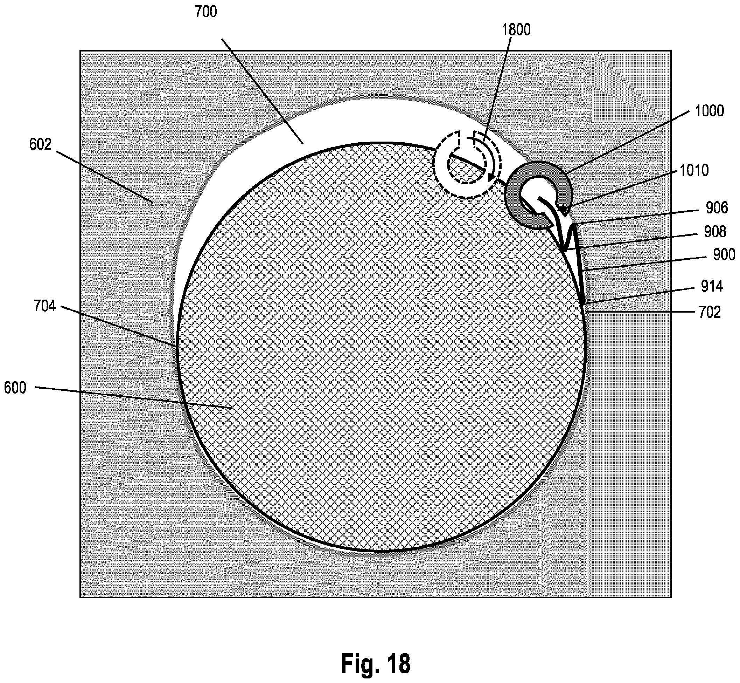

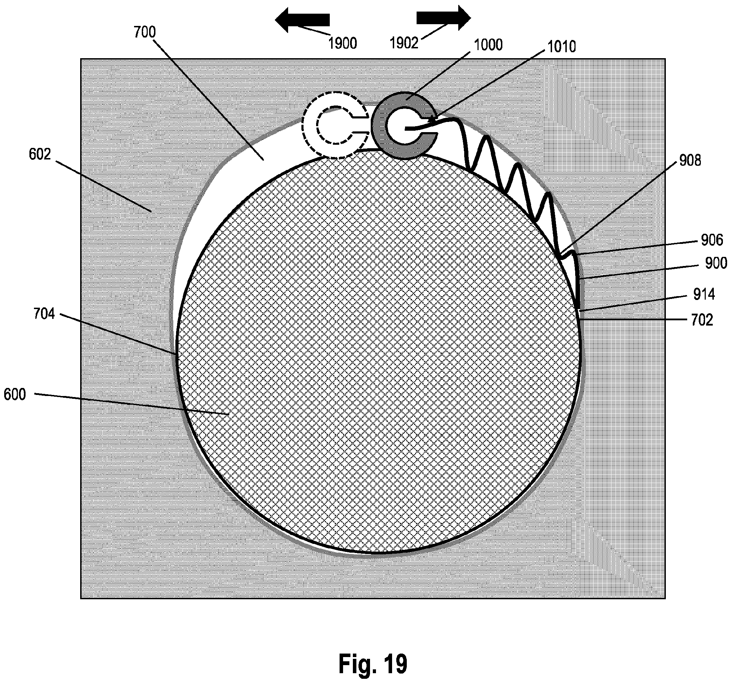

Fig. 18 illustrates the deployment of the elongate wire 900 within a non-circular gap between a therapeutic implant 890 and the neighboring tissue, according to some aspects of the present disclosure. In Fig. 18 (and Figs 19-21B described below), the therapeutic implant 890 is an occlusion device 600 disposed within the opening of the LAA 602, as shown in Fig. 7C. Although Figs 18-21B illustrate a wire 900 being inserted into a gap between an occlusion device 600 and a wall of the LAA 602, it should be understood that similar device, systems, and processes apply to other types of therapeutic implants 890 and tissues. For example, similar devices, systems, and methods may be applied to a paravalvular leakage between a valve replacement and neighboring heart wall (as shown in Figs 2-5B).

Returning to Fig. 18, in some aspects, the non-circular gap 700 may be crescent-shaped with a first end or cusp 702 and a second end or cusp 704. The wire 900 may be initially inserted at the first cusp 702, then inserted along the gap 700 to the second cusp 704.

When the catheter 1000 is delivered to the site of the gap 700, the catheter 1000 may not be disposed at the desired orientation or location. For example, the initial position of the catheter 1000 may be shown with dotted lines. In the initial position, the catheter 1000 is not located proximate the first cusp 702 of the gap 700. Moreover, the notch 1010 is oriented upward or away from the first cusp 702.

Thus, the catheter 1000 may be moved and rotated (as shown by arrow 1800) into a deployment position as shown by solid lines. In the deployment position, the catheter 1000 may be located closer to the first cusp 702 and the notch 1010 may be oriented so that it faces the first cusp 702. As illustrated in Fig. 18, a portion of the wire 900 has been inserted into the gap 700 such that a first end 914 of the wire 900 is disposed at the first cusp 702 of the gap 700. The wire 900 the serpentine shape 904 is oriented so that the peaks 906 contact the LAA 602 and the troughs 908 contact the occlusion device 600. In other aspects, the troughs 908 contact the LAA 602 and the peaks 906 contact the occlusion device 600.

The wire 900 is implanted into the gap 700 so that the wire 900 maintains the serpentine shape 904. In some aspects, the amplitude 910 of the serpentine shape 904 may be constrained within the gap 700 so that the amplitude of the deployed wire 900 matches the width of the gap 700, as described in more detail below.

As shown in Fig. 18, the wire 900 is deployed generally parallel to the gap 700 through the notch 1010 so that the serpentine shape 904 can be better inserted into the gap 700. Thus, the peaks 906 and troughs 908 can be oriented along the ends of the gap 700 to better fill it.

Fig. 19 the continued deployment of the elongate wire 900 within a non-circular gap between a therapeutic implant 890 and the neighboring tissue, according to some aspects of the present disclosure. As compared to Fig. 18, the catheter 1000 in Fig. 19 has been moved further along the gap 700 towards the second cusp 704. Fig. 19 may illustrate the wire 900 inserted into approximately half of the gap 700. A first intermediate position of the catheter 1000 may be shown by solid lines. A second intermediate position closer to the second cusp 704 may be shown by dotted lines. The catheter 1000 may be moved from the first intermediate position to the second intermediate position (as shown by arrow 1900) to continue deploying the wire 900 into the gap 700. The wire 900 can continue to be inserted into the gap 700 until the second end 916 of the wire 900 is inserted at the second cusp 704. In some aspects, multiple wires 900 are inserted into the gap 700. In some aspects, the catheter 1000 cuts the wire 900 to form the second end 916 when the catheter 1000 reaches the second cusp 704.

In some aspects, the amplitude 910 of the serpentine shape 904 may be constrained within the gap 700 so that the amplitude of the deployed wire 900 matches the width of the gap 700, as described in more detail below.

Moreover, because the serpentine shape 904 of the wire 900 is constrained by the LAA 602 and occlusion device 600 on either side of the gap 700, an outward force (as shown by arrow 1902) is applied by the wire 900 on the LAA 602 and occlusion device 600. As a result, the LAA 602 and occlusion device 600 may also apply an inward force (as shown by arrow 1900) on the wire 900. The flexible and/or resilient serpentine shape 904 may transfer this force along the gap 700 towards the catheter 1000. Thus, this force may move or push the catheter 1000 to the left towards the second cusp 704 (as shown by arrow 1900).

In other aspects, the catheter 1000 may be moved from the second cusp 704 to the first cusp 702 and, thus, from the second intermediate position to the first intermediate position (as shown by arrow 1902). Thus, the first end 914 of the wire 900 may be inserted at the second cusp 704 and the second end 916 of the wire 900 may be inserted at the first cusp 702.

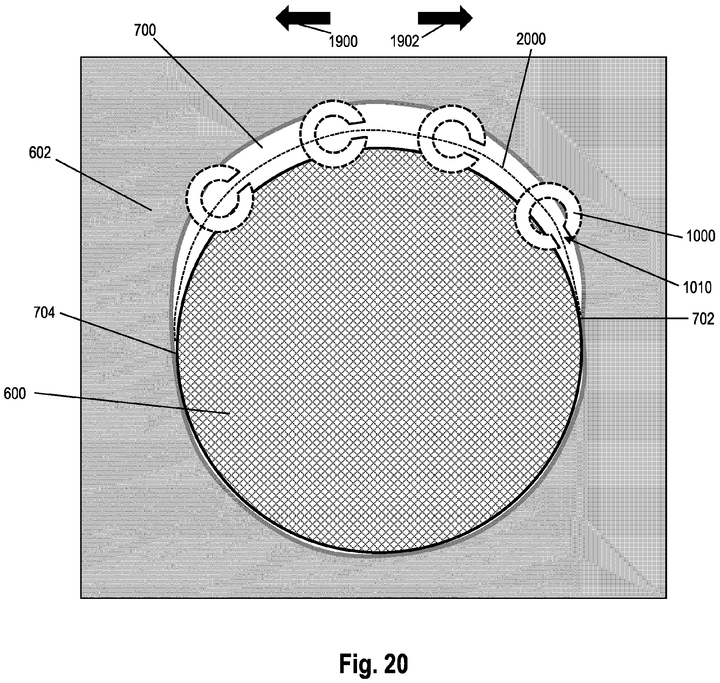

Fig. 20 illustrates various positions of the catheter 1000 as it is moved from the first cusp 702 to the second cusp 704 of the gap 700, according to some aspects of the present disclosure. As the catheter 1000 is moved along the gap 700, the notch 1010 may be oriented and reoriented so that it continually faces a centerline 2000 of the gap 700 as the catheter 1000 is moved between the first cusp 702 and second cusp 704 (as shown by arrow 1900). As the wire 900 is deployed out of the notch 1010 of the catheter 1000, the wire 900 may expand towards its heat-set serpentine shape 904. Thus, even as the notch 1010 faces the centerline 2000, the wire 900 will be deployed such that the serpentine shape 904 is oriented so that the peaks 906 contact the LAA 602 and the troughs 908 contact the occlusion device 600 (as shown in Figs 18-19) or vice versa as the wire 900 moves towards its heat-set serpentine shape 904. In some aspects, the notch 1010 may be oriented in the opposite direction shown in Fig. 20 and the catheter 1000 may be moved along the centerline from the second cusp 704 to the first cusp 702 along the centerline 2000 (as shown by arrow 1902). In some aspects, the contact between the wire 900 on the one hand and the LAA 602 and/or occlusion device 600 causes/urges the catheter 1000 to move and/or reorient itself (as described with respect to Fig. 19) into the positions and orientations shown in Fig. 20.

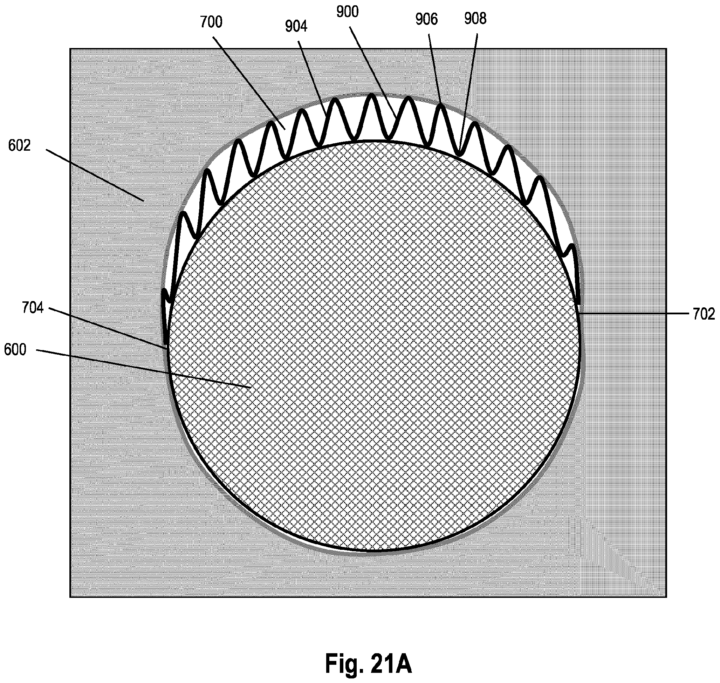

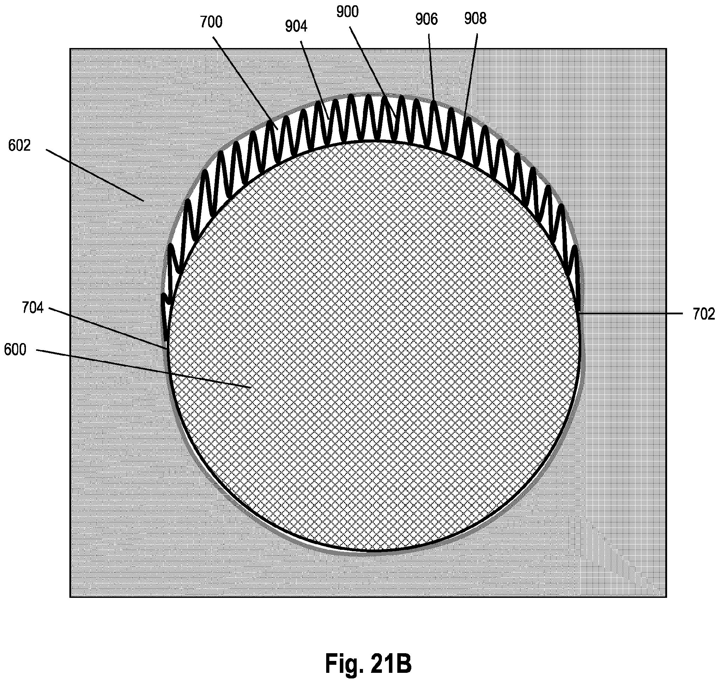

Figs 21A-21B illustrate the wire 900 inserted into the gap 700 such that the gap 700 is filled, according to some aspects of the present disclosure. Fig. 21A illustrates the wire 900 inserted into the gap 700 in a relatively less compressed state and Fig. 21B illustrates the wire 900 inserted into the gap 700 in a relatively more compressed state. As illustrated in both Figs 21A-21B, the wire 900 maintains a serpentine or sinusoidal shape 904 (i.e. a regular shape) when it is inserted into the gap 700. A first end 914 of the wire 900 is inserted at the first cusp 702 and the second end 916 is inserted at the second cusp 704. In some aspects, the serpentine shape 904 is oriented so that the peaks 906 contact the LAA 602 and the troughs 908 contact the occlusion device 600 (or vice versa).

In some aspects, the width of the gap 700 (i.e. the distance between the occlusion device 600 and the LAA 602) may vary between the first cusp 702 and second cusp 704. As the wire 900 is inserted into the gap, the amplitude may be constrained by the width. Thus, the amplitude of the serpentine shape 904 may vary between the first and second cusp 702, 704 to match the width. This may allow the wire 900 to better fit to the non-circular shape of the gap 700.

When the occlusion device 600 and LAA 602 constrain the amplitude of the serpentine shape 904, the wire 900 is inserted into the gap 700 under tension. This tension maintains the wire 900 within the gap 700. For example, this may generate a frictional force between the wire 900 and the occlusion device 600 and the LAA 602, which thereby maintains the wire 900 within the gap 700.

In some aspects, the serpentine shape 904 may allow the wire 900 to function like a compression spring that is biased outward. Thus, when the wire 900 is inserted into the gap 700, the ends 914, 916 of the wire 900 are biased outward, applying an outward force to the cusps 702, 704 of the gap 700. This outward force on the cusps 702, 704 may push the cusps outward thereby narrowing or closing the gap 700 along its length.

A difference between Fig. 21A and 21B is that the distance between the peak and troughs (comparable to the "wavelength") is larger in Fig. 21A than it is in Fig. 21B. Thus, the wire 900 shown in Fig. 21B may be more compressed into the gap 700 (i.e. in a more compressed state) and, thus, may be under more tension than the wire 900 in Fig. 21A. In some aspects, more wire 900 (i.e. a longer total length of wire) may be inserted in the gap 700 in Fig. 21B as compared to Fig. 21A. Thus, the wire 900 in Fig. 21B may cover a larger area of the gap 700 than the relatively less compressed wire 900 (i.e. in a less compressed state) in Fig. 21A. In some aspects, the wire 900 in Fig. 21B better prevent leakage or be maintained better within the gap 700 than the wire 900 in Fig. 21A by covering a larger area of the gap 700 and by applying more outward force on the cusps 702, 704 to make the gap 700 more narrow.

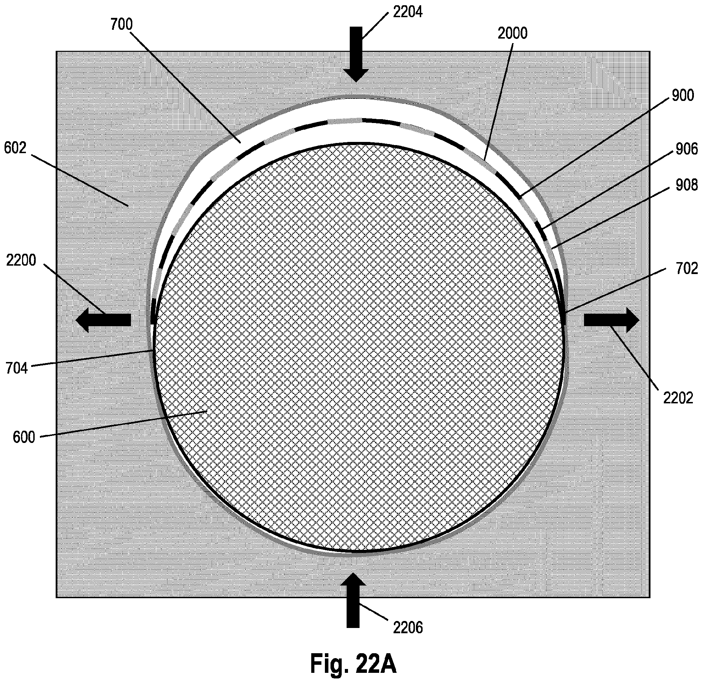

Figs 22A-22B illustrate another example of a wire 900 inserted into the gap 700 such that the gap 700 is narrowed and/or closed, according to some aspects of the present disclosure. In this example, the wire 900 has been inserted such that the peaks 906 are oriented towards the top of the gap 700 (out of the page) and the troughs 908 are oriented towards the bottom of the gap 700 (into the page). In some aspects, the wire 900 is rotated 90 degrees relative to the wire 900 inserted into the gap 700 in Figs 21A-21B. The position/orientation of the wire 900 in Figs. 22A-22B can be described as perpendicular to the gap 700, whereas the position/orientation of the wire 900 in Figs. 21A-2B can be described as parallel to the gap 700.

Fig. 22A illustrates the gap 700 just after the wire 900 has been inserted therein. In some aspects, the wire 900 may be inserted along the centerline 2000, as shown in Fig. 20. In other aspects, the wire 900 may not be inserted along the centerline 2000. For example, the wire 900 may be inserted along the perimeter of the occlusion device 600 or along the perimeter of the LAA 602 from the first cusp 702 to the second cusp 704 of the gap 700 (or vice versa). The plane of the bent wire 900 (into and out of plane of page), which is formed by the center axis of the wire as it forms the sinusoidal pattern, can follow the circumference/annulus of the occlusion device 600 as it goes at least partially around the circumference/annulus of the occlusion device 600.

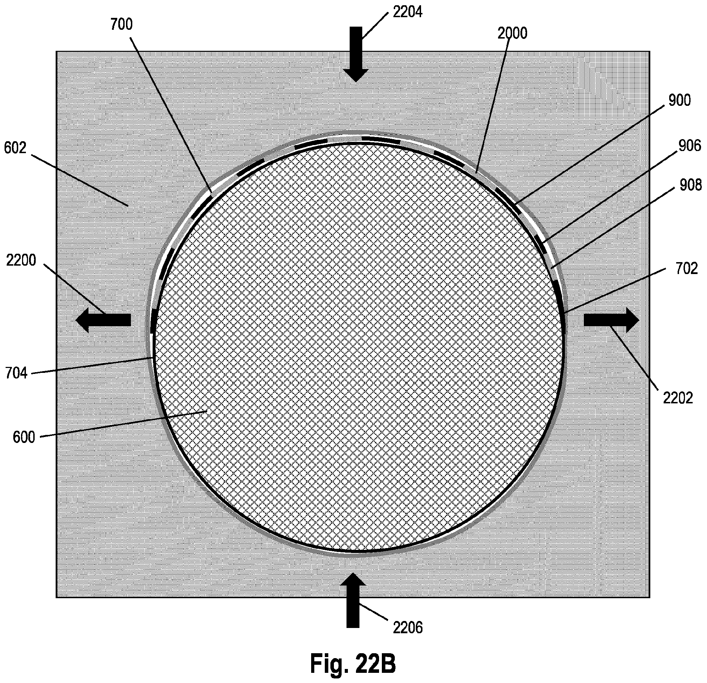

Fig. 22B illustrates the gap 700 after the wire 900 has narrowed and/or closed the gap 700. When wire 900 is inserted into the gap 700, the serpentine shape 904 of the wire 900 may be biased outward to apply a force or press outward on the cusps 702, 704 (as shown by arrows 2200, 2202) like a compression spring. By pressing outward on the cusps 702, 704, the wire 900 may apply a force to the LAA 602 and/or occlusion device 600 such that the LAA 602 moves inward towards the centerline 2000 (as shown by arrow 2204) and/or the occlusion device 600 moves inward towards the centerline (as shown by arrow 2206). When the LAA 602 and/or the occlusion device 600 moves inward, the gap 700 may narrow and/or close. Thus, after the wire 900 is inserted as shown in Fig. 22A, the gap 700 may narrow and/or close as shown in Fig. 22B. In some aspects, the gap 700 may narrow and/or close during insertion of the wire 900. The wire 900 will endothelialize, such that tissue grows to fill any remaining space between the wire 900, the occlusion device 600 and/or the LAA 602.

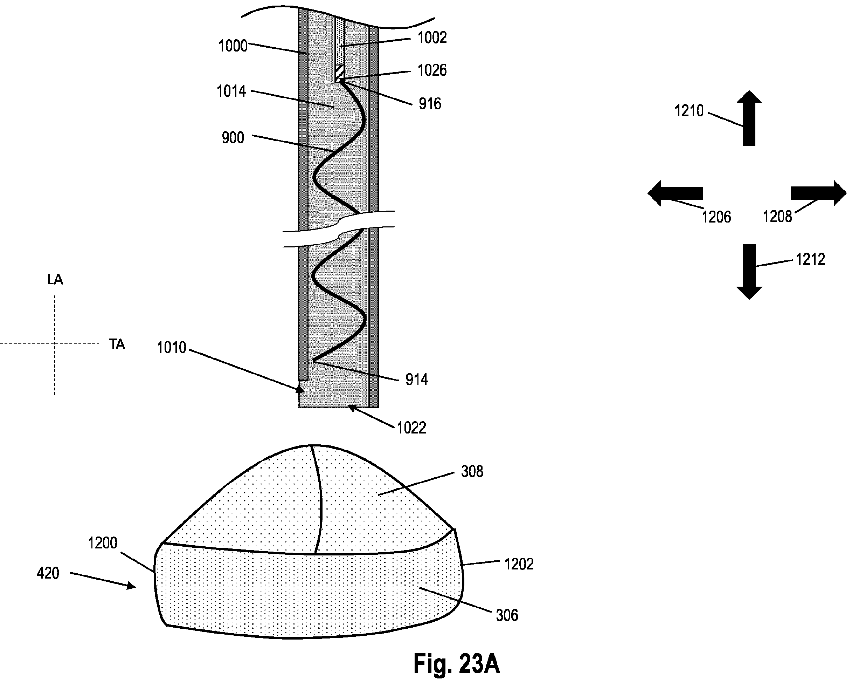

Figs 23A-23D illustrate various steps in a method of deploying and implanting the elongate wire 900 using a wire delivery catheter 1000 and a push rod 1002. Any suitable wire 900 described herein may be implanted according to the illustrated method, including those described in reference to Figs 9-11B. Any suitable wire delivery catheter 1000 described herein may be used to deliver and/or deploy the wire 900 according to the illustrated method, including those described in reference to Figs 12A-17B. Any suitable push rod 1002 described herein may be used to deliver and/or deploy the wire 900 according to the illustrated method, including those described in reference to Figs 15-17B.

In the illustrated embodiment, the wire 900 is inserted into a gap 420 around a replacement valve 300 (as shown in Figs 2-5B). However, it should be understood that the method may be used to fill gaps around any therapeutic implant 890. For example, a similar method may be used to insert a wire 900 into a gap 700 around an occlusion device 600 (as shown in Figs 6-7C). Figs 23A-23C illustrate a side view of the replacement valve 300 having leaflets 308 and a replacement valve wall or annulus 306. The replacement valve 300 is illustrated in Figs 23A-23C from the perspective of an observer positioned within the gap 700, looking toward the replacement valve 300, with the native tissue (e.g., heart wall 210) behind the observer. The catheter 1000 is shown in cross-section along the 22-22 line in Fig. 12A (which is the same as the 15-15, 16-16, and 17-17 line) so that the push rod 1002 and wire 900 within the lumen 1006 are visible. The position/orientation of the wire 900 in Figs. 23A-23D can be described as perpendicular to the gap 700.

Fig. 23A illustrates a diagrammatic view of the elongate wire 900 being delivered to the gap 420.

In some aspects, the replacement valve 300 may have been previously implanted into the patient during a separate procedure. For example, the replacement valve 300 may have been implanted between one day and six months before the current method is performed (i.e. the procedure to prevent leakage through the gap 420). In these aspects, a physician may notice (e.g. during a follow up appointment or check-up) that the replacement valve 300 has not been performing properly and/or has a leak. Thus, the physician may decide to perform the current method to treat, fill, and/or close the gap 420 to improve functioning of the replacement valve 300. In some aspects, any degree of endothelialization may exist around the replacement valve 300 before the current method is performed such that one or more endothelial cells are disposed on the replacement valve 300 and/or tissue.

In other aspects, the replacement valve 300 may be implanted during the same procedure as the current method is performed. In these aspects, a physician may notice a leak or gap 420 between the replacement valve 300 and the tissue after the replacement valve 300 has been deployed. Thus, the physician may implement the current methods before the procedure is completed to treat, fill, and/or close the gap 420 so that the replacement valve 300 will perform properly.

As shown in Fig. 12A, the catheter 1000 may be moved proximate to the gap 420 such that the distal opening 1022 is oriented towards the gap 420. The LA of the catheter 1000 may be perpendicular to the gap 420. Moreover, the notch 1010 may be oriented along the centerline of the gap 420 (i.e. parallel to the perimeter of the replacement valve 300 and/or tissue). As illustrated in Figs 23A-23D, a first end 1200 of the gap 420 is disposed to the left and the second end 1202 of the gap 420 is disposed to the right. Thus, the notch 1010 may be generally located towards a first end 1200 of the gap 420.

The push rod 1002 may be disposed within the catheter 1000 such that the distal end 1026 contacts, couples, and/or attaches to the first end 916 of the wire 900, as shown in Fig. 16. The wire 900 may be disposed in the catheter 1000 such that the first end 914 is pointed proximally (as shown by proximal arrow 1210) relative to the catheter 1000 and the second end 916 is pointed distally (as shown by distal arrow 1212) towards the distal opening 1022 of the catheter 1000, as shown in Fig. 16. As described above, the inner surface 1014 of the catheter 1000 may apply a force to wire 900 in this position so that the amplitude 910 of the wire 900 is constrained to the inner diameter 1016. Thus, the wire 900 may be constrained or flexed such that it is spring-loaded within the lumen 1006 of the catheter 1000.

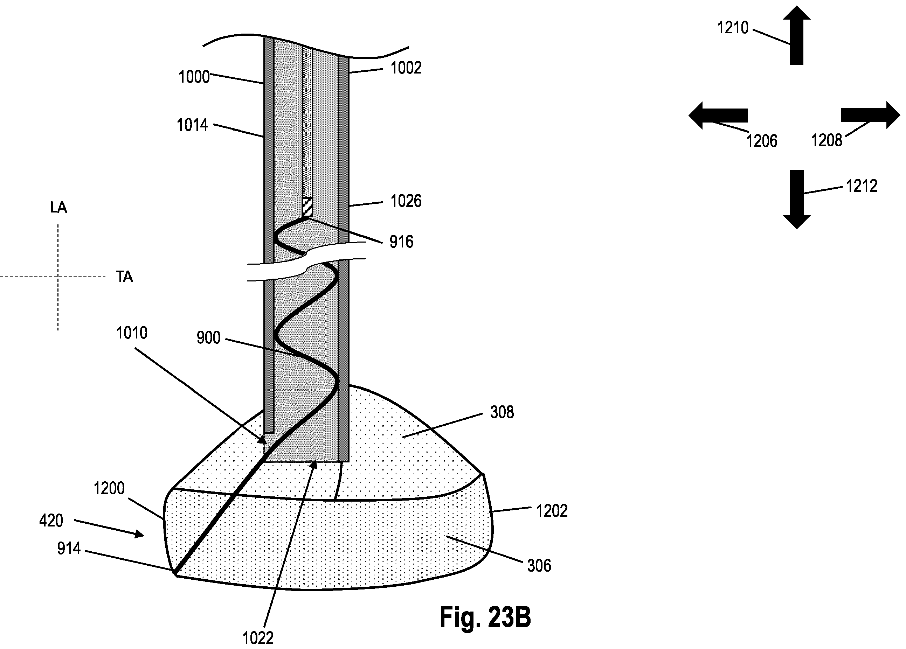

Fig. 23B illustrates a diagrammatic view of the catheter 1000 and push rod 1002 moving the first end 914 of the wire 900 out of notch 1010 of the catheter 1000 and into the gap 420. In some aspects, the push rod 1002 may be moved distally (as shown by arrow 1212) towards the gap 420 to move or push the first end 914 of the wire 900 out of the notch 1010 of the catheter 1000.

The wire 900 may be pushed out of the notch 1010 of the catheter 1000 generally perpendicularly relative to the LA of the catheter 1000. Thus, the wire 900 may be pushed out so that it is generally parallel to the gap 420.

In some aspects, when the first end 914 of the wire 900 is pushed out of the notch 1010 of the catheter 1000, the portion of the wire 900 outside of the catheter 1000 becomes unconstrained by the inner surface 1014 of the catheter 1000. Thus, the wire 900 may move towards its heat-set serpentine shape 904, which may have a larger amplitude 910 than the inner diameter 1016 of the catheter 1000. Thus, as the wire 900 is pushed out of the catheter 1000, the first end 914 of the wire 900 contacts the first end 1200 of the gap 420.

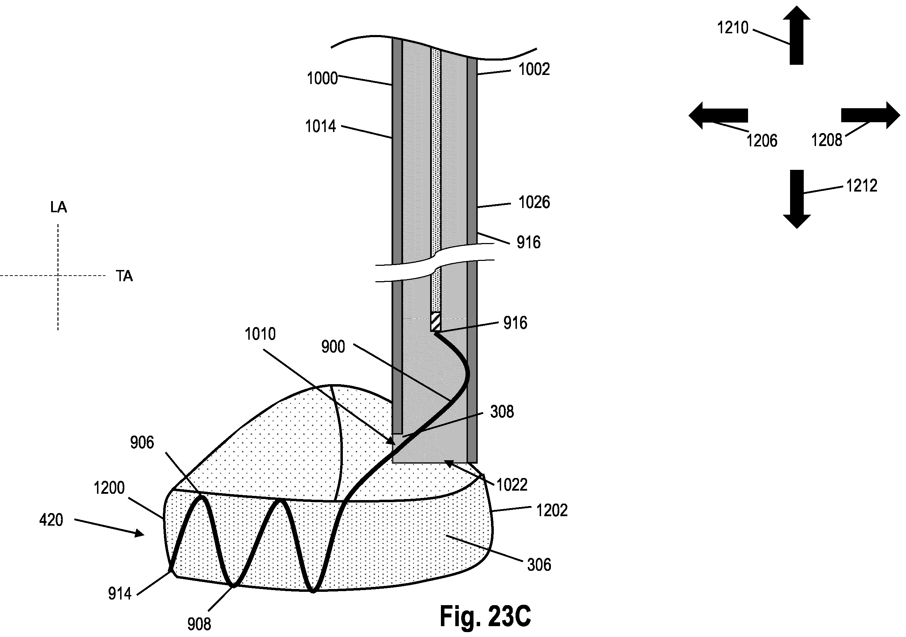

Fig. 23C illustrates a diagrammatic view of the catheter 1000 and push rod 1002 moving a larger portion of the wire 900 out of notch 1010 of the catheter 1000 and into the gap 420. The push rod 1002 may continue to be moved distally (as shown by arrow 1212) towards the gap 420 to continue moving or pushing the wire 900 out of the notch 1010 of the catheter 1000.

The serpentine shape 904 of the wire 900 may be oriented so that the peaks 906 are oriented upward (as shown by arrow 1210) and the troughs 908 are oriented downward (as shown by arrow 1212) (or vice versa). In some aspects, as the push rod 1002 pushes the wire 900 out of the catheter 1000, the serpentine shape 904 presses against the first end 1200 of the gap 420 (as shown by arrow 1206). In response, the first end 1200 of the gap 420 presses against the wire 900 (as shown by arrow 1208), thereby moving the catheter 1000 towards the right along the gap 420 towards the second end 1202 (as shown by arrow 1208).

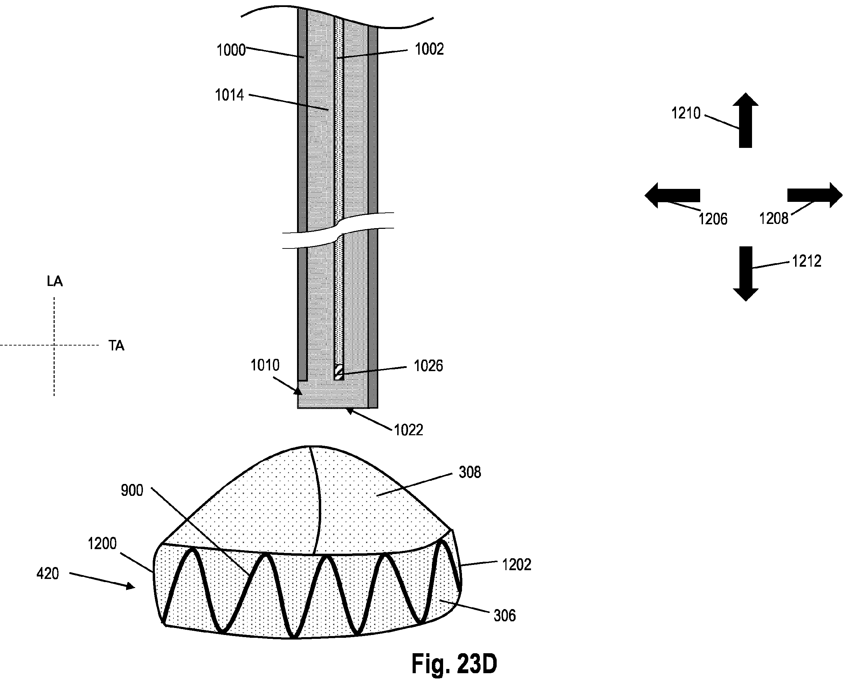

Fig. 23D illustrates a diagrammatic view of the wire 900 after it has been completely pushed out of the catheter 1000 and is implanted into the gap 420. As more of the wire 900 has pushed out, the serpentine shape 904 may be compressed or under tension between the first end 1200 and the second end 1202 of the gap 420. As described above, the ends 914, 916 of the serpentine shape 904 of the wire 900 may press against the ends 1200, 1202 of the gap 420 to narrow and/or close the gap 420. The plane of the bent wire 900 (within the plane of page), which formed by the center axis of the wire as it forms the sinusoidal pattern, can follow the circumference/annulus of the replacement valve 300 as it goes at least partially around the circumference/annulus of the replacement valve 300.

Once the second end 916 of the wire 900 is pushed out of the catheter 1000, the distal end 1026 of the push rod 1002 may stop contacting the second end 916 of the wire 900. If the distal end 1026 includes a coupling element, the coupling element may decouple or detach from the second end 916 of the wire 900.

Once the push rod 1002 stops contacting, disengages, or detaches from the wire 900, the catheter 1000 and push rod 1002 may be moved away from the gap 420 (as shown by arrow 1210). In some aspects, the push rod 1002 may be moved proximally and may be removed from the catheter 1000 so that a new elongate wire 900 can be engaged with the push rod 1002 and inserted into the catheter 1000. Thus, another elongate wire 900 can be delivered to and deployed at the current gap 420 or a new gap (e.g. 410) along the replacement valve 300. Once all of the desired wires 900 have been deployed, the catheter 1000 and push rod 1002 may be removed from the patient. In other aspects, the catheter 1000 and push rod 1002 may be removed completely and a new catheter 1000, push rod 1002, and wire 900 may be inserted into the patient to deliver subsequent wires 900. In some aspects, multiple wires 900 may be disposed within the catheter 1000 so that multiple wires 900 can be inserted into the gap or gaps at a time, which may advantageously decrease the cost and/or time of performing the procedure.

In some aspects, the serpentine shape 904 of the wire 900 is 3D or extends in all dimensions (x, y, and z dimensions, as shown in Figs 9, 11B, and 11C). Thus, as the wire 900 is implanted, the wire 900 may extend along the width of the gap (between the therapeutic device 890 and the tissue) as well as along the length of the gap (between the ends of the gap) and the thickness of the gap (from a bottom to a top of the therapeutic implant 890). Thus, in some aspects, a single wire 900 may be implanted to fill the gap. In some aspects, multiple wires 900 may be stacked on top of each other within the gap so that the gap is filled along the thickness of the gap.

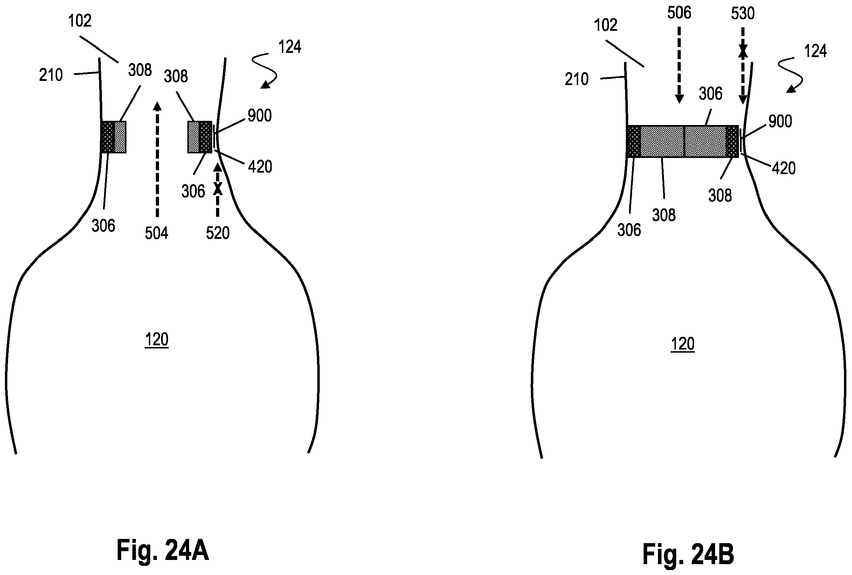

Figs 24A-24B illustrate a diagrammatic cross-sectional side view of a replacement valve 300 shown in Figs 5A-5B after a gap has been closed using one or more elongate wires 900. Fig. 24A illustrates the replacement valve 300 with the valve leaflets 308 open to allow blood to flow from the left ventricle 120 to the aorta 102 (i.e. during systole), indicated by directional arrow 504. Because the wire 900 has been implanted into the gap 420, the wire 900 has narrowed and/or closed the gap 420 between the replacement valve 300 and the heart wall 210. Thus, during systole, blood flows through the opening of the replacement valve 300 (as shown by arrow 504) but there is no blood flow around the outside of the replacement valve 300 along the heart wall 210 (as shown by arrow 520).

Fig. 23B illustrates the replacement valve 300 with the valve leaflets 308 closed (i.e. during diastole) to prevent mitral regurgitation (i.e. blood flow from the aorta 102 to the left ventricle as shown by arrow 506). Because the gap 420 has been narrowed and/or closed using the wire 900, there is no blood flow between the outside of the replacement valve 300 and the heart wall 210 (as shown by arrow 530). Therefore, the deployment of the wire 900 may prevent paravalvular leakage around the replacement valve 300, which may allow the valve to function properly.

The wire 900 inserted into the gap 420 in Figs 23A-23B is 2D as shown in Fig. 11A. Thus, the peaks 906 are oriented upwards towards the top of the gap 420 and the troughs 908 are oriented downwards towards the bottom of the gap 420 (or vice versa). The position/orientation of the wire 900 in Figs. 24A-24B can be described as perpendicular to the gap 700. However, the 3D wire 900 illustrated in Figs 11B-11C may alternatively be inserted into the gap 420 to prevent leakage therethrough. Moreover, multiple wires may be inserted such that they are stacked vertically between the top and bottom of the gap 420. The wire 900 will endothelialize, such that tissue grows to fill any remaining space between the wire 900, the replacement valve 300 and/or the native tissue 210.

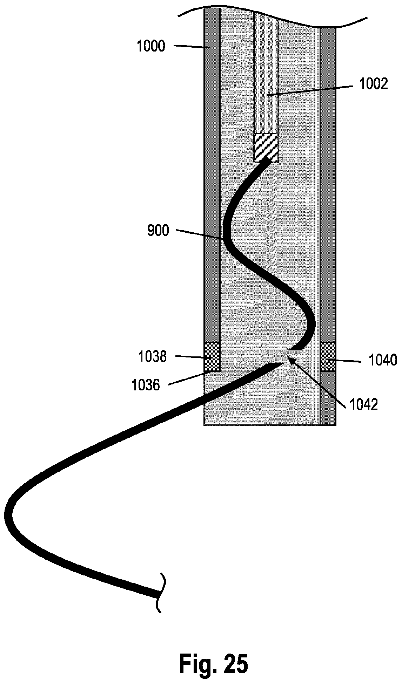

Fig. 25 illustrates an example of a catheter 1000 with a push rod 1002 and wire 900 disposed therein, according to some aspects of the present disclosure. In this example, the catheter 1000 includes a cutter 1036 that is configured to cut the wire 900 at a particular point. In some aspects, the cutter may use electrodes to cut the wire 900. For example, a first electrode 1038 may be disposed on a first side of the catheter 1000 and a second electrode 1040 may be disposed on a second side of the catheter 1000 opposite the first electrode 1038. The electrodes 1038, 1040 may be activated such that a current is passed across the lumen 1006 of the catheter 1000. Thus, the part of the wire 900 disposed between the electrodes may be heated such that the wire 900 is cut.

In other aspects, the cutter 2400 may be a mechanical cutter. For example, the mechanical cutter may have one or more sharp or serrated blades that are capable of cutting the wire 900. However, the mechanical cutter may use any suitable shape or part that is configured to cut the wire 900.

Thus, the physician may activate the cutter 1036 to cut the wire 900 at a particular point. The portion of the wire 900 distal to the cut 1042 may be inserted into the gap. The portion of the wire 900 proximal to the cut 1042 may remain in the catheter 1000 and may be either inserted into the gap at the delivery site, into a different gap, or may be removed from the body. This may advantageously allow the physician to insert a desired amount of wire 900 within the gap instead of using a preset amount of wire 900 that may not perfectly conform to the shape and/or size of the gap.

Fig. 26 is a schematic diagram of a processor circuit 1600, according to aspects of the present disclosure. The processor circuit 1600 may be implemented in the processing system 810, the system 800, or other devices or workstations (e.g., third-party workstations, network routers, etc.), or on a cloud processor or other remote processing unit, as necessary to implement the method. As shown, the processor circuit 1600 may include a processor 1610, a memory 1612, and a communication module 1614. These elements may be in direct or indirect communication with each other, for example via one or more buses.

The processor 1610 may include a central processing unit (CPU), a digital signal processor (DSP), an ASIC, a controller, or any combination of general-purpose computing devices, reduced instruction set computing (RISC) devices, application-specific integrated circuits (ASICs), field programmable gate arrays (FPGAs), or other related logic devices, including mechanical and quantum computers. The processor 1610 may also comprise another hardware device, a firmware device, or any combination thereof configured to perform the operations described herein. The processor 1610 may also be implemented as a combination of computing devices, e.g., a combination of a DSP and a microprocessor, a plurality of microprocessors, one or more microprocessors in conjunction with a DSP core, or any other such configuration.

The memory 1612 may include a cache memory (e.g., a cache memory of the processor 1610), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and non-volatile memory, or a combination of different types of memory. In an aspect, the memory 1612 includes a non-transitory computer-readable medium. The memory 1612 may store instructions 1616. The instructions 1616 may include instructions that, when executed by the processor 1610, cause the processor 1610 to perform the operations described herein. Instructions 1616 may also be referred to as code. The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer-readable statement or many computer-readable statements.

The communication module 1614 can include any electronic circuitry and/or logic circuitry to facilitate direct or indirect communication of data between the processor circuit 1600, and other processors or devices. In that regard, the communication module 1614 can be an input/output (I/O) device. In some instances, the communication module 1614 facilitates direct or indirect communication between various elements of the processor circuit 1600 and/or the system 800. The communication module 1614 may communicate within the processor circuit 1600 through numerous methods or protocols. Serial communication protocols may include but are not limited to United States Serial Protocol Interface (US SPI), Inter-Integrated Circuit (I²C), Recommended Standard 232 (RS-232), RS-485, Controller Area Network (CAN), Ethernet, Aeronautical Radio, Incorporated 429 (ARINC 429), MODBUS, Military Standard 1553 (MIL-STD-1553), or any other suitable method or protocol. Parallel protocols include but are not limited to Industry Standard Architecture (ISA), Advanced Technology Attachment (ATA), Small Computer System Interface (SCSI), Peripheral Component Interconnect (PCI), Institute of Electrical and Electronics Engineers 488 (IEEE-488), IEEE-1284, and other suitable protocols. Where appropriate, serial and parallel communications may be bridged by a Universal Asynchronous Receiver Transmitter (UART), Universal Synchronous Receiver Transmitter (USART), or other appropriate subsystem.

External communication (including but not limited to software updates, firmware updates, preset sharing between the processor and central server, or readings from the leaflet puncture and slitting device) may be accomplished using any suitable wireless or wired communication technology, such as a cable interface such as a universal serial bus (USB), micro USB, Lightning, or FireWire interface, Bluetooth, Wi-Fi, ZigBee, Li-Fi, or cellular data connections such as 2G/GSM (global system for mobiles), 3G/UMTS (universal mobile telecommunications system), 4G, long term evolution (LTE), WiMax, or 5G. For example, a Bluetooth Low Energy (BLE) radio can be used to establish connectivity with a cloud service, for transmission of data, and for receipt of software patches. The controller may be configured to communicate with a remote server, or a local device such as a laptop, tablet, or handheld device, or may include a display capable of showing status variables and other information. Information may also be transferred on physical media such as a USB flash drive or memory stick.

As will be readily appreciated by those having ordinary skill in the art after becoming familiar with the teachings herein, the wire 900 advantageously permits a clinician (e.g., a heart surgeon) to repair leaks that may form during a therapeutic implant 890 (e.g. a replacement valve 300 or an occlusion device 600). Although primarily intended for the repair of leakage of therapeutic implants 890 for the heart, the same technology could be applied to any implant or device where leakage occurs.

The logical operations making up the aspects of the technology described herein are referred to variously as operations, steps, objects, elements, components, or modules. Furthermore, it should be understood that these may be arranged or performed in any order, unless explicitly claimed otherwise or a specific order is inherently necessitated by the claim language. It should further be understood that the described technology may be employed in single-use and multi-use devices for medical or nonmedical use.

All directional references e.g., upper, lower, inner, outer, upward, downward, left, right, lateral, front, back, top, bottom, above, below, vertical, horizontal, clockwise, counterclockwise, proximal, and distal are only used for identification purposes to aid the reader's understanding of the claimed subject matter, and do not create limitations, particularly as to the position, orientation, or use of aspects of the present disclosure. Connection references, e.g., attached, coupled, connected, and joined are to be construed broadly and may include intermediate members between a collection of elements and relative movement between elements unless otherwise indicated. As such, connection references do not necessarily imply that two elements are directly connected and in fixed relation to each other. The term "or" shall be interpreted to mean "and/or" rather than "exclusive or." The word "comprising" does not exclude other elements or steps, and the indefinite article "a" or "an" does not exclude a plurality. Unless otherwise noted in the claims, stated values shall be interpreted as illustrative only and shall not be taken to be limiting.

The above specification, examples and data provide a complete description of the structure and use of exemplary aspects of the present disclosure, e.g., as defined in the claims. Although various aspects of the claimed subject matter have been described above with a certain degree of particularity, or with reference to one or more individual aspects, those skilled in the art could make numerous alterations to the disclosed aspects without departing from the spirit or scope of the claimed subject matter.

Still other aspects are contemplated. It is intended that all matter contained in the above description and shown in the accompanying drawings shall be interpreted as illustrative only of particular aspects and not limiting. Changes in detail or structure may be made without departing from the basic elements of the subject matter as defined in the following claims.

## Claims

1. An apparatus, comprising:
an elongate wire configured to be implanted within a heart of a patient such that the elongate wire is positioned within a non-circular gap between a therapeutic implant positioned within the heart and a native heart tissue of the patient to stop a leakage of blood through the non-circular gap,
wherein the elongate wire comprises a single length of material set in a serpentine shape, and
wherein the elongate wire is configured to retain the serpentine shape when the elongate wire is positioned within the non-circular gap.

2. The apparatus of claim 1,
wherein the serpentine shape comprises a plurality of peaks and a plurality of troughs,
wherein, when a first length of the elongate wire is positioned within the non-circular gap, the serpentine shape comprises a first state with a first distance between at least one of the plurality of peaks or the plurality of troughs, and
wherein the first state comprises an uncompressed state or a relatively less compressed state.

3. The apparatus of claim 2,
wherein, when a greater, second length of the elongate wire is positioned within the non-circular gap, the serpentine shape comprises a second state with a smaller, second distance between at least one of the plurality of peaks or the plurality of troughs, and
wherein the second state comprises a compressed state.

4. The apparatus of claim 1, wherein, when the elongate wire is positioned within the non-circular gap, the elongate wire is configured to have compression in a longitudinal direction of the serpentine shape.

5. The apparatus of claim 1, wherein elongate wire comprises at least one of fibers or barbs configured to facilitate endothelization.

6. The apparatus of claim 1, further comprising:
a delivery catheter comprising a catheter wall defining a lumen configured to receive the elongate wire before the elongate wire is positioned within the non-circular gap; and
a push rod configured to be positioned within the lumen, contact the elongate wire within the lumen, and have distal movement causing the elongate wire to exit the delivery catheter.

7. The apparatus of claim 6,
wherein the delivery catheter comprises a longitudinal axis, a proximal portion, and a distal portion,
wherein the distal portion comprises a longitudinal slit on a side of the catheter wall, wherein the serpentine shape is configured to cause the elongate wire to exit the delivery catheter via the longitudinal slit at an angle relative to the longitudinal axis, wherein preferably the serpentine shape is configured to such that contact between the elongate wire and at least one of the therapeutic implant or the native heart tissue is configured to urge the delivery catheter to move from a first end of the non-circular gap to a second end of the non-circular gap.

8. A system for treating leakage between an implanted therapeutic device and tissue of a patient, comprising:
a catheter comprising a lumen and a distal portion, wherein the distal portion comprises a notch; and
an implant comprising a wire comprising a serpentine shape along at least a part of a length of the wire,
wherein the implant is disposed within the lumen of the catheter, and
wherein the implant is configured to exit the lumen via the notch at the distal portion of the catheter.

9. The system of claim 8, wherein the serpentine shape of the wire comprises a plurality of peaks and a plurality of troughs.

10. The system of claim 9,
wherein each peak of the plurality of peaks is spaced from neighboring troughs of the plurality of troughs in an x-dimension and a y-dimension,
wherein each peak of the plurality of peaks is spaced from neighboring peaks along the x-dimension, and
wherein each trough of the plurality of troughs is spaced from neighboring peaks along the x-dimension.

11. The system of claim 8, wherein the implant further comprises at least one of a plurality of barbs or a plurality of fibers disposed on at least a portion of the wire.

12. The system of claim 8, wherein the wire is compressible via the serpentine shape such that the wire is compressed within the lumen of the catheter.

13. The system of claim 8, wherein the notch extends proximally from a distal end of the distal portion of the catheter.

14. The system of claim 8, wherein the notch extends from an outer surface of the catheter to the lumen.

15. The system of claim 8, further comprising a push rod configured to move the implant distally within the lumen such that the implant exits the lumen through the notch in the distal portion.
